# EUROPEAN PATENT APPLICATION

(11) **EP 4 296 232 A1**
(43) Date of publication of application: **27.12.2023**
(21) Application number: 22756321.0
(22) Date of filing: 21.02.2022
(51) Int. Cl.: C01G 9/02, A61K 8/27, A61K 8/891, A61K 8/894, A61Q 17/04

(54) **SURFACE-MODIFIED ZINC OXIDE PAPRTICLES, DISPERSION LIQUID, COSMETIC PREPARATION, AND METHOD FOR PRODUCING SURFACE-MODIFIED ZINC OXIDE PARTICLES**

(30) Priority: 22.02.2021 JP 2021026315; 22.02.2021 JP 2021026403
(71) Applicant: Sumitomo Osaka Cement Co., Ltd., Tokyo, 105-8641 (JP)
(72) Inventor: SUMA Syunsuke, Tokyo 102-8465 (JP)
(74) Representative: Becker, Eberhard
(86) International application number: PCT/JP2022/006902
(87) International publication number: WO 2022/177004

(57) **Abstract**

Surface-modified zinc oxide particles of the present invention are surface-modified zinc oxide particles in which the particle surfaces of zinc oxide particles are treated with a hydrolyzable surface treatment agent, in which the color difference ΔE before and after irradiation with simulated sunlight is 4.0 or lower.

## Description

### Technical Field

The present invention relates to surface-modified zinc oxide particles, a method for producing the same, a dispersion liquid, and a cosmetic preparation.

### Background Art

It is known that zinc oxide has excellent ultraviolet shielding properties, high gas barrier properties, and, furthermore, high transparency. Therefore, particles made of zinc oxide as a forming material (hereinafter, referred to as "zinc oxide particles") have functions of ultraviolet shielding, gas barrier, or the like and are used as a forming material of various materials that require transparency. Examples of such materials include ultraviolet shielding films, ultraviolet shielding glass, cosmetic preparations, gas barrier films, and the like.

As a method for improving the transparency of the above-described various materials, for example, a method for reducing the primary particle diameters of zinc oxide particles, which are a forming material, is exemplified. As a method for reducing the primary particle diameters of zinc oxide particles, various methods such as a thermal decomposition method and a vapor phase method are being studied (for example, refer to Patent Literatures 1 and 2).

In a case where zinc oxide particles are applied to cosmetic preparations, a surface treatment is performed on the zinc oxide particles with a surface treatment agent in order to adapt the surfaces of the zinc oxide particles to the properties of cosmetic products or suppress the catalytic activity of the zinc oxide particles.

In the following description, zinc oxide particles that have been surface-treated with a surface treatment agent and have the surface treatment agent on the surfaces will be referred to as surface-modified zinc oxide particles.

Such surface-modified zinc oxide particles are blended into cosmetic preparations as they are or blended into cosmetic preparations in a state of a dispersion liquid in which the zinc oxide particles are dispersed in a dispersion medium.

In the case of being blended into oily cosmetic preparations, emulsion-type oil phases, or the like, zinc oxide particles surface-treated with a silane coupling agent or the like having an alkoxy group are used (for example, refer to Patent Literatures 3 and 4).

### Citation List

### Patent Literature

[Patent Literature No. 1] Japanese Laid-open Patent Publication No. 2002-284527
[Patent Literature No. 2] Japanese Laid-open Patent Publication No. 2000-95519
[Patent Literature No. 3] Pamphlet of International Publication No. WO2017/130632
[Patent Literature No. 4] Japanese Laid-open Patent Publication No. 2007-51188

### Summary of Invention

### Technical Problem

However, there has been a demand for additional improvement in the ultraviolet shielding properties of surface-modified zinc oxide particles.

The present invention has been made in view of the above circumstances, and an object of the present invention is to provide surface-modified zinc oxide particles having excellent ultraviolet shielding properties and a method for producing the same. In addition, another object of the present invention is to provide a dispersion liquid and a cosmetic preparation that contain such surface-modified zinc oxide particles.

### Solution to Problem

That is, surface-modified zinc oxide particles of a first aspect of the present invention are surface-modified zinc oxide particles in which particle surfaces of zinc oxide particles are treated with a hydrolyzable surface treatment agent, in which a color difference ΔE before and after irradiation with simulated sunlight is 4.0 or lower.

The surface-modified zinc oxide particles of the first aspect preferably include the following characteristics. It is also preferable to combine two or more of the following characteristics.

In the surface-modified zinc oxide particles of the first aspect of the present invention, a total light transmittance at a wavelength of 360 nm is preferably 48% or lower.

In the surface-modified zinc oxide particles of the first aspect of the present invention, a total light transmittance at a wavelength of 360 nm is preferably 40% or lower.

In the surface-modified zinc oxide particles of the first aspect of the present invention, the transmittance may be a value before exposure to the following environment.

In the surface-modified zinc oxide particles of the first aspect of the present invention, a total light transmittance at a wavelength of 360 nm after exposure to an environment of 85°C and 95 %RH for 48 hours is preferably 40% or lower.

In the surface-modified zinc oxide particles of the first aspect of the present invention, a content of the surface treatment agent is preferably 4% by mass or more and 20% by mass or less with respect to a total of the surface treatment agent and the zinc oxide particles.

In the surface-modified zinc oxide particles of the first aspect of the present invention, the surface treatment agent is preferably a hydrolyzed silane coupling agent having an alkoxy group.

A dispersion liquid of a second aspect of the present invention contains the surface-modified zinc oxide particles and a dispersion medium.

A cosmetic preparation according to a third aspect of the present invention contains the surface-modified zinc oxide particles and a cosmetic base raw material.

A method for producing surface-modified zinc oxide particles according to a fourth aspect of the present invention is a method for producing the surface-modified zinc oxide particles, having a first step of mixing a surface treatment agent and water to prepare a hydrolysate and a second step of mixing the hydrolysate obtained in the first step and zinc oxide particles to prepare a first mixture.

The production method of the fourth aspect preferably includes a step of drying the first mixture obtained in the second step.

In the production method of the fourth aspect, it is also preferable that a step of mixing the hydrolysate obtained in the first step and the mixture obtained in the step is repeated once or more.

### Advantageous Effects of Invention

According to the present invention, it is possible to provide surface-modified zinc oxide particles having excellent ultraviolet shielding properties. In addition, according to the present invention, it is possible to provide a dispersion liquid and a cosmetic preparation that contain such surface-modified zinc oxide particles.

### Description of Embodiments

Embodiments of the surface-modified zinc oxide particles of the present invention, the method for producing the same, the dispersion liquid, and the cosmetic preparation will be described.

The present embodiments are simply specific descriptions for better understanding of the gist of the invention and do not limit the present invention unless particularly otherwise described. Numerical values, amounts, materials, types, times, temperatures, orders, and the like can be changed, omitted, replaced, added, and the like in the present invention within the scope of the gist. A first embodiment and a second embodiment may share expressions, preferable characteristics or conditions unless otherwise described.

### [First embodiment]

### [Surface-modified zinc oxide particles]

Surface-modified zinc oxide particles of the present embodiment are surface-modified zinc oxide particles in which the particle surfaces of zinc oxide particles are treated with a hydrolyzable surface treatment agent, in which the color difference ΔE before and after irradiation with simulated sunlight is 4.0 or lower.

The color difference ΔE is preferably 3.5 or lower, more preferably 3.0 or lower, still more preferably 2.5 or lower, and particularly preferably 2.0 or lower.

The lower limit value of the color difference ΔE is 0, and may be 0.01, may be 0.05, or may be 0.1.

The surface-modified zinc oxide particles of the present embodiment have a color difference ΔE of 4.0 or lower and thus have excellent ultraviolet shielding properties.

The color difference ΔE before and after irradiation with simulated sunlight in the present embodiment means, for example, a color difference measured by the following measurement method.

3 g of t-butyl methoxydibenzoylmethane, 3 g of polyhydroxystearic acid, and 94 g of tri(caprylic acid/capric acid) glyceryl are mixed, and a solution in which t-butyl methoxydibenzoylmethane is completely dissolved is produced.

Next, 2.0 g of the obtained solution and 3.0 g of surface-modified zinc oxide particles are mixed with a kneader, for example, a kneader (manufactured by THINKY Corporation, model number: ARE-310) to become uniform, thereby obtaining a liquid mixture.

Next, the obtained liquid mixture is sandwiched between assembled quartz cells for a spectrophotometer, for example, quartz cells (manufactured by GL Sciences Inc., model number: AB20-UV-05), so that the optical path length becomes 0.5 mm, thereby producing an assembled cell.

Next, the obtained assembled cell is set in an ultraviolet-visible-near-infrared spectrophotometer, for example, an ultraviolet-visible-near-infrared spectrophotometer (manufactured by JASCO Corporation, model number: V-770), the diffuse reflectance spectrum of the liquid mixture is measured using an integrating sphere, and the L₁*, a₁*, b₁* of the liquid mixture is calculated from the measurement results.

Next, the assembled cell containing the liquid mixture is irradiated with simulated sunlight having an accumulated light intensity of 300 kJ/m² using a xenon lamp of a light irradiation test device, for example, a small light irradiation test device (manufactured by Iwasaki Electric Co., Ltd., model number: EYE SUN-CUBE Xenon).

The irradiated assembled cell is set again in the ultraviolet-visible-near-infrared spectrophotometer, the diffuse reflectance spectrum is measured in the same manner as described above, and the L₂*, a₂*, b₂* of the liquid mixture after irradiation with simulated sunlight is calculated from the measurement results.

Next, the color difference ΔE = ((L₂* - L₁*)² + (a₂* - a₁*)² + (b₂* - b₁*)²)^{1/2} is calculated.

The L₁*, a₁*, b₁ and the *L₂*, a₂*, b₂* are values in the L*a*b* colorimetric chromaticity diagram.

The present measurement method is a test for evaluating the surface treatment state of the surface-modified zinc oxide particles of the present embodiment using simulated sunlight. In the light irradiation test device, a xenon light source that is close to sunlight is used. Therefore, the irradiation with the xenon lamp of the present measurement device can be regarded as being equivalent to exposure to actual sunlight. An influence of ultraviolet rays equivalent to exposure to actual sunlight for approximately 10 minutes can be evaluated by the irradiation with simulated sunlight having an accumulated light intensity of 300 kJ/m² in the present measurement method. As a result of measuring the color differences ΔE of surface-modified zinc oxide particles produced in examples, the measurement results of the color difference ΔE did not change significantly between the irradiation time of 10 minutes and the irradiation time of 3 hours. Therefore, in the present measurement method, the color difference was evaluated with an accumulated light intensity of 300 kJ/m², which can be regarded as the irradiation time with sunlight of approximately 10 minutes.

The measurement method in the present embodiment will be described.

t-Butyl methoxydibenzoylmethane is an organic ultraviolet absorber that is commonly blended into cosmetic preparations intended to shield ultraviolet rays. Polyhydroxystearic acid is a dispersant and is added in the present measurement to uniformly mix the surface-modified zinc oxide particles and t-butyl methoxydibenzoylmethane in a solvent. Tri(caprylic acid/capric acid) glyceryl is a common solvent that is used in cosmetic preparations.

When the mixture containing the surface-modified zinc oxide particles and t-butyl methoxydibenzoylmethane is exposed to simulated sunlight, if the surface treatment is not sufficient, t-butyl methoxydibenzoylmethane is altered due to the photocatalytic activity of the zinc oxide particles or the elution of zinc ions, and the mixture discolors.

The present inventors and the like found that, if a surface treatment is performed on the zinc oxide particles so that the color difference ΔE becomes 4.0 or lower, the deterioration of the ultraviolet shielding properties of the zinc oxide particles is suppressed even when the zinc oxide particles are exposed to sunlight and completed the present invention. In addition, the present inventors and the like found that surface-modified zinc oxide particles having a color difference ΔE of 4.0 or lower can be obtained by hydrolyzing a hydrolyzable surface modifier in advance and then performing the surface treatment.

In the present embodiment, the reason for the ultraviolet shielding properties of the surface-modified zinc oxide particles to be improved when the color difference ΔE becomes 4.0 or lower is not clear. The reason for the color difference ΔE to become small is presumed that the photocatalytic activity or the elution of zinc ions is suppressed. However, there is no method for directly measuring how the hydrolyzable surface modifier modifies the surfaces of the zinc oxide particles. Therefore, it is not clear that how the previously-hydrolyzed surface modifier surface-modifies the surfaces of the zinc oxide particles leads to a decrease in the color difference ΔE. The reason is also presumed that the surface modifier is uniformly and densely present on the surfaces of the zinc oxide particles, which is not enough to describe everything.

In the present embodiment, the effect of the use of the previously-hydrolyzed surface treatment agent decreasing the color difference ΔE to 4.0 or lower and enabling the improvement in the ultraviolet shielding properties of the surface-modified zinc oxide particles is presumed to develop due to a number of factors intricately intertwined. Specifically, the effect is presumed to develop due to a number of factors intricately intertwined such as the shapes, specific surface area, and particle size distribution of the zinc oxide particles, the degree of hydrolysis of the hydrolyzable surface modifier, the attachment condition of the hydrolyzable surface modifier to the zinc oxide particles, and the attachment rate of the hydrolyzable surface modifier to the zinc oxide particles. Therefore, it is considered that it is almost impossible to directly specify the characteristics of the surface-modified zinc oxide particles of the present embodiment with the state of the surfaces of the zinc oxide particles surface-modified with the hydrolyzable surface treatment agent that has been hydrolyzed in advance.

As a result of various studies, the present inventors and the like focused on the color difference ΔE and found that the ultraviolet shielding properties of surface-modified zinc oxide particles are improved by surface-treating the zinc oxide particles with a previously-hydrolyzed surface treatment agent so that the color difference ΔE becomes 4.0 or lower.

In the surface-modified zinc oxide particles of the present embodiment, the total light transmittance at a wavelength of 360 nm measured by the following measurement method is preferably 48% or lower, more preferably 45% or lower, still more preferably 40% or lower, and far still more preferably 35% or lower. For example, the total light transmittance may be 43% or lower, 38% or lower, 30% or lower, or the like.

The values may mean values measured without performing an accelerated test to be described below. For example, the values may be values measured immediately after the production or storage by a common method of the surface-modified zinc oxide particles.

A cosmetic preparation having excellent ultraviolet shielding properties can be obtained by blending in surface-modified zinc oxide particles having a total light transmittance at a wavelength of 360 nm, that is, a sum of the linear transmittance and the diffuse transmittance, of 48% or lower.

The lower limit of the total light transmittance at a wavelength of 360 nm may be 0%, may be 1%, may be 10%, or may be 20%.

In the surface-modified zinc oxide particles of the present embodiment, the linear transmittance at a wavelength of 550 nm measured by the following measurement method is preferably 80% or higher, more preferably 85% or higher, and still more preferably 90% or higher.

A cosmetic preparation having excellent transparency can be obtained by blending in surface-modified zinc oxide particles having a linear transmittance at a wavelength of 550 nm of 80% or higher.

The upper limit of the linear transmittance at a wavelength of 550 nm may be 100%, may be 99%, or may be 98%.

In the present embodiment, the total light transmittance at a wavelength of 360 nm and the linear transmittance at a wavelength of 550 nm may mean, for example, values measured by the following measurement method.

10 g of the surface-modified zinc oxide particles, 88 g of cyclopentasiloxane (for example, manufactured by Dow Toray Co., Ltd., model number: DOWSIL SH 245 Fluid), and 2 g of polyglyceryl-3 polydimethylsiloxyethyl dimethicone (for example, manufactured by Shin-Etsu Chemical Co., Ltd., model number: KF-6106) are mixed to obtain a dispersion liquid.

Next, a dispersion treatment is performed on this liquid mixture at 9500 rpm for 5 minutes using a homogenizer, for example, a homogenizer (manufactured by IKA, ULTRA-TURRAX (registered trademark) series: T25 basic) to obtain a dispersion liquid for evaluation.

The obtained dispersion liquid for evaluation is diluted with cyclopentasiloxane such that the concentration of the surface-modified zinc oxide particles becomes 0.005% by mass. This diluted liquid is put into a quartz cell having an optical path length of 10 mm, and the total light transmittance at 360 nm and the linear transmittance at 550 nm are measured using an ultraviolet-visible-near-infrared spectrophotometer, for example, an ultraviolet-visible-near-infrared spectrophotometer (manufactured by JASCO Corporation, model number: V-770).

In the surface-modified zinc oxide particles of the present embodiment, the particle diameter (d90) when the cumulative volume percentage of the particle size distribution measured by the following measurement method is 90% is preferably 1 µm or lower, more preferably 0.85 µm or lower, still more preferably 0.5 µm or lower, far still more preferably 0.4 µm or lower, and particularly preferably 0.3 µm or lower.

A cosmetic preparation having excellent transparency and ultraviolet shielding properties can be obtained by blending surface-modified zinc oxide particles having d90 of 1 µm or lower into the cosmetic preparation.

The lower limit of d90 may be 0.05 µm or may be 0.1 µm.

d90 of the surface-modified zinc oxide particles of the present embodiment may mean, for example, a value measured by the following measurement method.

10 g of the surface-modified zinc oxide particles, 88 g of cyclopentasiloxane (for example, manufactured by Dow Toray Co., Ltd., model number: DOWSIL SH 245 Fluid), and 2 g of polyglyceryl-3 polydimethylsiloxyethyl dimethicone (for example, manufactured by Shin-Etsu Chemical Co., Ltd., model number: KF-6106) are mixed to obtain a dispersion liquid.

Next, a dispersion treatment is performed on this liquid mixture at 9500 rpm for 5 minutes using a homogenizer, for example, a homogenizer (manufactured by IKA, ULTRA-TURRAX (registered trademark) series: T25 basic) to obtain a dispersion liquid for evaluation.

The obtained dispersion liquid for evaluation is diluted with cyclopentasiloxane such that the content of the surface-modified zinc oxide particles becomes 0.1% by mass. This diluted liquid is used to measure the particle diameter (d90) when the cumulative volume percentage of the particle size distribution is 90% using a dynamic light scattering particle size distribution analyzer, for example, a dynamic light scattering particle size distribution analyzer (manufactured by MicrotracBEL Corp., model number: NANOTRAC WAVE). The particle diameter (d10) when the cumulative volume percentage of the particle size distribution is 10% and the particle diameter (d50) when the cumulative volume percentage of the particle size distribution is 50% can also be measured.

In the surface-modified zinc oxide particles of the present embodiment, the total light transmittance at a wavelength of 360 nm measured by the above-described measurement method after the accelerated test where the surface-modified zinc oxide particles are exposed to an environment of 85°C and 95 %RH for 48 hours may be 49% or lower, 48% or lower, or 45% or lower, but is particularly preferably 40% or lower. The total light transmittance may be 38% or lower or 30% or lower as necessary. A cosmetic preparation having particularly excellent temporal stability can be obtained by blending surface-modified zinc oxide particles having a total light transmittance at a wavelength of 360 nm after the accelerated test of 40% or lower into the cosmetic preparation.

The lower limit of the total light transmittance at a wavelength of 360 nm after the accelerated test may be 0%, may be 1%, may be 10%, or may be 20%.

In the surface-modified zinc oxide particles of the present embodiment, the linear transmittance at a wavelength of 550 nm after the accelerated test where the surface-modified zinc oxide particles are exposed to an environment of 85°C and 95 %RH for 48 hours may be 83% or higher, 85% or higher, or 88% or higher, but is particularly preferably 90% or higher. The linear transmittance may be, for example, 93% or higher or may be 95% or higher. A cosmetic preparation having excellent temporal stability can be obtained by blending surface-modified zinc oxide particles having a linear transmittance at a wavelength of 550 nm after the accelerated test of 90% or higher into the cosmetic preparation.

The upper limit of the linear transmittance at a wavelength of 550 nm after the accelerated test may be 100%, may be 99%, or may be 98%.

In the surface-modified zinc oxide particles of the present embodiment, d90 after the accelerated test where the surface-modified zinc oxide particles are exposed to an environment of 85°C and 95 %RH for 48 hours is preferably 0.80 µm or less, more preferably 0.75 µm or less, still more preferably 0.6 µm or less, far still more preferably 0.5 µm or less, even far still more preferably 0.4 um or less, and particularly preferably 0.3 µm or less. A cosmetic preparation having particularly excellent temporal stability can be obtained by blending surface-modified zinc oxide particles having d90 after the accelerated test of 0.3 µm or less into the cosmetic preparation.

The lower limit of d90 may be 0.05 µm or may be 0.1 µm.

The specific surface area of the surface-modified zinc oxide particles of the present embodiment can be arbitrarily selected, but is preferably 1.5 m²/g or more, more preferably 2.5 m²/g or more, and still more preferably 4 m²/g or more. In addition, the specific surface area of the surface-modified zinc oxide particles may be, for example, 65 m²/g or less and is preferably 55 m²/g or less, and more preferably 50 m²/g or less. The specific surface area of the surface-modified zinc oxide particles may be 45 m²/g or less, may be 40 m²/g or less, or may be 20 m²/g or less as necessary. The upper limit value and lower limit value of the specific surface area of the surface-modified zinc oxide particles can be arbitrarily combined together.

When the specific surface area of the surface-modified zinc oxide particles is 1.5 m²/g or more and 65 m²/g or less, transparency and ultraviolet shielding properties are excellent in a case where the surface-modified zinc oxide particles are blended into a cosmetic preparation.

In a case where there is a desire to enhance the transparency in the case of blending the surface-modified zinc oxide particles into a cosmetic preparation, the specific surface area of the surface-modified zinc oxide particles is preferably 8 m²/g or more, more preferably 15 m²/g or more, and still more preferably 20 m²/g or more.

For example, the specific surface area of the surface-modified zinc oxide particles is preferably 20 m²/g or more and 65 m²/g or less, more preferably 20 m²/g or more and 60 m²/g or less, and still more preferably 20 m²/g or more and 55 m²/g or less. The specific surface area may be 20.0 m²/g or more and 30.0 m²/g or less, 20.0 m²/g or more and 38.0 m²/g or less, or 20.0 m²/g or more and 50.0 m²/g or less as necessary. In a case where the specific surface area of the surface-modified zinc oxide particles is the lower limit value or higher, it is possible to obtain a cosmetic preparation having excellent transparency when the surface-modified zinc oxide particles are blended into the cosmetic preparation. On the other hand, when the specific surface area of the surface-modified zinc oxide particles is the upper limit value or lower, since the surface energy of the particles is not too large, it is possible to blend the surface-modified zinc oxide particles into a cosmetic preparation with a small amount of energy.

Incidentally, in a case where there is a desire to enhance the ultraviolet shielding properties in the UVA region in the case of blending the surface-modified zinc oxide particles into a cosmetic preparation, the specific surface area of the surface-modified zinc oxide particles is preferably less than 20 m²/g, more preferably 15 m²/g or less, and still more preferably less than 8 m²/g.

For example, the specific surface area of the surface-modified zinc oxide particles is preferably 1.5 m²/g or more and less than 20 m²/g, more preferably 1.5 m²/g or more and 15 m²/g or less, and still more preferably 1.5 m²/g or more and less than 8 m²/g. In a case where the specific surface area of the surface-modified zinc oxide particles is the lower limit value or higher, it is possible to obtain a cosmetic preparation having transparency when the surface-modified zinc oxide particles are blended into the cosmetic preparation. On the other hand, when the specific surface area of the surface-modified zinc oxide particles is less than the upper limit value, since the surface energy of the particles is not too large, it is possible to blend the surface-modified zinc oxide particles into a cosmetic preparation with a small amount of energy and to obtain a cosmetic preparation having excellent ultraviolet shielding properties in the UVA region.

The specific surface area (unit: m²/g) of the surface-modified zinc oxide particles in the present embodiment is the BET specific surface area obtained by the BET method.

Examples of a method for measuring the specific surface area of the surface-modified zinc oxide particles include a BET method in which a full automatic specific surface area-measuring instrument (for example, trade name: Macsorb HM Model-1201, manufactured by Mountech Co., Ltd.) is used.

In the present embodiment, "being treated with a surface treatment agent" refers to the fact that the surface treatment agent comes into contact with or bonds to the zinc oxide particles by an interaction therebetween. As the contact, for example, physical adsorption is exemplified. In addition, as the bond, for example, an ionic bond, a hydrogen bond, a covalent bond, and the like are exemplified.

The amount of the surface treatment agent in the surface-modified zinc oxide particles may be appropriately adjusted in accordance with the specific surface area of the zinc oxide particles and the degree of hydrophobilization of a cosmetic preparation into which the surface-modified zinc oxide particles are to be blended before use. For example, the amount of the surface treatment agent in the surface-modified zinc oxide particles is preferably 1% by mass or more and 20% by mass or less, more preferably 5% by mass or more and 18% by mass or less, and still more preferably 8% by mass or more and 16% by mass or less of the total mass of the zinc oxide particles and the surface treatment agent. When the amount of the surface treatment agent is the lower limit value or more, it is possible to blend hydrophilic zinc oxide particles into an oily cosmetic preparation. In addition, when the amount of the surface treatment agent is the upper limit value or less, the ultraviolet shielding properties of the zinc oxide particles when blended into a cosmetic preparation are sufficiently exhibited.

From the viewpoint of decreasing the color difference ΔE and improving the temporal stability, the content of the surface treatment agent is preferably 4% by mass or more and 20% by mass or less, more preferably 6% by mass or more and 18% by mass or less, and still more preferably 8% by mass or more and 16% by mass or less of the total mass of the zinc oxide particles and the surface treatment agent.

In a case where the surface treatment agent is a silane coupling agent, the content of the surface treatment agent in the surface-modified zinc oxide particles can be calculated by, for example, quantitatively analyzing the amount of Si in the surface-modified zinc oxide particles with an inductively coupled plasma atomic emission spectrometer.

### "Zinc oxide particles"

The specific surface area of the zinc oxide particles (before the surface treatment) of the present embodiment can be arbitrarily selected, but is preferably 1.5 m²/g or more, more preferably 2.5 m²/g or more, and still more preferably 4 m²/g or more. In addition, the specific surface area of the zinc oxide particles is preferably 65 m²/g or less and more preferably 55 m²/g or less. The specific surface area of the zinc oxide particles may be 50 m²/g or less, may be 40 m²/g or less, or may be 20 m²/g or less as necessary.

The specific surface area of the zinc oxide particles before the surface treatment and the specific surface area of the surface-modified zinc oxide particles slightly vary depending on how to attach the silane coupling agent, but do not change significantly.

Therefore, in order to obtain surface-modified zinc oxide particles having a desired specific surface area, zinc oxide particles having a desired specific surface area needs to be used. That is, it is possible for the surface-modified zinc oxide particles of the present embodiment to preferably have the same value or range as the above-described preferable value or range of the specific surface area of the zinc oxide particles.

b* in the L*a*b* colorimetric chromaticity diagram of the zinc oxide particles before the treatment is preferably 10 or lower. When zinc oxide particles having b* in the above range are used, b* of the surface-modified zinc oxide particles can also become 4.0 or higher and 18 or lower, and b* of the surface-modified zinc oxide particles becomes larger than b* of the zinc oxide particles before the surface treatment. As a result, it is possible to suppress the surface-modified zinc oxide particles to yellowness at which the surface-modified zinc oxide particles can be applied to cosmetic preparations. The lower limit value of b* of the zinc oxide particles is not particularly limited and may be 0, may be 1.0, or may be 1.5.

### "Hydrolyzable surface treatment agent"

The hydrolyzable surface treatment agent of the present embodiment is not particularly limited as long as the surface treatment agent is hydrolyzable and can be used in cosmetic preparations. "Being hydrolyzable" in the present embodiment includes not only the hydrolysis of an alkoxy group but also the dealkalization of metal soaps or the dehydrogenation of SiH of silicone.

Examples of such a surface treatment agent include at least one selected from the group consisting of a silane compound, a silicone compound, a fatty acid, a fatty acid soap, a fatty acid ester, and an organic titanate compound.

In addition, a surfactant may also be used.

Examples of the silane compound that is used for the surface treatment include alkylsilane, fluoroalkylsilane, and the like.

Examples of the alkylsilane include methyltrimethoxysilane, ethyltrimethoxysilane, hexyltrimethoxysilane, octyltrimethoxysilane, octyltriethoxysilane, and the like.

Examples of the fluoroalkylsilane include trifluoromethylethyltrimethoxysilane, heptadecafluorodecyltrimethoxysilane, and the like.

Among these silane compounds, alkylsilane is preferable, and octyltriethoxysilane is particularly preferable.

These silane compounds may be used singly or two or more silane compounds may be used in combination.

Examples of the silicone compound include silicone oil, methicone, dimethicone, hydrogen dimethicone, triethoxysilylethyl polydimethylsiloxyethyl dimethicone, triethoxysilylethyl polydimethylsiloxyethylhexyl dimethicone, (acrylate/tridecyl acrylate/triethoxysilylpropyl methacrylate/dimethicone methacrylate) copolymers, triethoxycaprylylsilane, and the like.

Examples of the silicone oil include methyl hydrogen polysiloxane, dimethyl polysiloxane, methylphenyl polysiloxane, and the like.

These silicone compounds may be used singly or two or more silicone compounds may be used in combination. In addition, as the silicone compound, a copolymer of these silicone compounds may also be used.

Examples of the fatty acid include palmitic acid, isostearic acid, stearic acid, lauric acid, myristic acid, behenic acid, oleic acid, rosin acid, 12-hydroxystearic acid, and the like.

Examples of the fatty acid soap include magnesium stearate, zinc stearate, aluminum stearate, calcium stearate, magnesium myristate, zinc myristate, aluminum distearate, aluminum dimyristate, aluminum 12-hydroxystearate, and the like.

Examples of the fatty acid ester include dextrin fatty acid esters, cholesterol fatty acid esters, sucrose fatty acid esters, starch fatty acid esters, and the like.

Examples of the organic titanate compound include isopropyl triisostearoyl titanate, isopropyl dimethacryl isostearoyl titanate, isopropyl tri(dodecyl) benzene sulfonyl titanate, neopentyl (diallyl)oxy tri(dioctyl) phosphate titanate, neopentyl (diallyl)oxy trineododecanoyl titanate, and the like.

The above-exemplified compounds may be used singly or two or more compounds may be used in combination.

Among the above-described surface treatment agents, a silane coupling agent having an alkoxy group is preferably used.

### "Silane coupling agent having alkoxy group"

The silane coupling agent having an alkoxy group can be arbitrarily selected, and preferable examples thereof include silane coupling agents represented by the following general formula (1) that can be used in cosmetic preparations.

R¹Si(OR²)₃ ··· (1)

(R¹ represents an alkyl group, fluoroalkyl group or phenyl group having 1 to 18 carbon atoms, and R² represents an alkyl group having 1 to 4 carbon atoms. R² may be the same as or different from one another.)

Such a silane coupling agent is preferably at least one selected from the group consisting of an alkylalkoxysilane, an allylalkoxysilane, a polysiloxane having an alkyl group in a side chain, and a polysiloxane having an allyl group in a side chain.

Examples of the alkylalkoxysilane include methyltrimethoxysilane, methyltriethoxysilane, methyltripropoxysilane, methyltributoxysilane, ethyltrimethoxysilane, ethyltriethoxysilane, ethyltripropoxysilane, ethyltributoxysilane, n-propyltrimethoxysilane, n-propyltriethoxysilane, n-propyltripropoxysilane, n-propyltributoxysilane, isopropyltrimethoxysilane, isopropyltriethoxysilane, isopropyltripropoxysilane, isopropyltributoxysilane, phenyltrimethoxysilane, phenyltriethoxysilane, phenyltripropoxysilane, phenyltributoxysilane, n-octyltrimethoxysilane, n-octyltriethoxysilane (triethoxycaprylylsilane), n-octadecyltrimethoxysilane, and the like.

As the silane coupling agent, it is also possible to use, for example, polymer-type silane coupling agents and the like having a siloxane skeleton as the main chain and having an alkoxy group and an acrylic group in the molecular structure such as dimethoxydiphenylsilane-triethoxycaprylylsilane crosspolymers, triethoxysilylethyl polydimethylsiloxyethylhexyl dimethicone, and triethoxysilylethyl polydimethylsiloxyethyl hexyl dimethicone.

As the silane coupling agent, it is also possible to use, for example, fluoroalkylalkoxysilanes and the like such as trifluoropropyltrimethoxysilane, perfluorooctyltriethoxysilane, and tridecafluorooctyltriethoxysilane.

These silane coupling agents may be used singly or two or more silane coupling agents may be mixed and used.

Among the above-described silane coupling agents, silane coupling agents having an octyl group in the molecule are more preferable. Specifically, silane coupling agents that are compatible with oil phases with a wide range of polarity, from natural oils or ester oils to silicone oils, are more preferable. As such silane coupling agents, at least one selected from the group consisting of n-octyltriethoxysilane, n-octyltrimethoxysilane, and a dimethoxydiphenylsilane-triethoxycaprylylsilane crosspolymer is particularly preferable.

These silane coupling agents may be used singly or two or more silane coupling agents may be mixed and used.

In the surface-modified zinc oxide particles of the present embodiment, the zinc oxide particles may be surface-treated using, in addition to the silane coupling agent, the surface treatment agent that is used for cosmetic preparations and is not a silane coupling agent as long as the characteristics of the surface-modified zinc oxide particles are not impaired.

According to the surface-modified zinc oxide particles of the present embodiment, the surface-modified zinc oxide particles are surface-modified zinc oxide particles in which the particle surfaces of zinc oxide particles are treated with a hydrolyzable surface treatment agent and the color difference ΔE before and after irradiation with simulated sunlight is 4.0 or lower and thus have excellent ultraviolet shielding properties.

### "Method for producing surface-modified zinc oxide particles"

A method for producing surface-modified zinc oxide particles according to the present embodiment is a method for producing the above-described surface-modified zinc oxide particles, including a first step of mixing a surface treatment agent and water to prepare a hydrolysate and a second step of mixing the hydrolysate and zinc oxide particles to prepare a first mixture. In addition, the method preferably has a step of drying the first mixture obtained in the second step.

In addition, in the method for producing surface-modified zinc oxide particles of the present embodiment, after the second step, a third step of mixing the first mixture obtained in the second step and the hydrolysate obtained in the first step to prepare a second mixture containing the first mixture and the hydrolysate may be performed. The method may have a step of drying the second mixture obtained in the third step after the third step. In the third step, the components may be mixed by adding the hydrolysate obtained in the first step to the first mixture obtained in the second step or by adding the first mixture obtained in the second step to the hydrolysate obtained in the first step.

The surface treatment of the present embodiment may be performed in a dry manner or in a wet manner. A main difference between the dry treatment and the wet treatment may be considered to be the amount of the solvent that is contained in the first mixture. In the present embodiment, a case where the solvent that is contained in the first mixture is less than 40% by mass will be described as the dry treatment, and a case where the solvent is 40% by mass or more will be described as the wet treatment.

### (Production method in dry manner)

A method in which the surfaces are treated in a dry manner will be described.

### "First step of preparing hydrolysate"

In the present step, a surface treatment agent and water are mixed to prepare a hydrolysate. The use of a hydrolysate in which at least a part of the surface treatment agent has been hydrolyzed in advance as described above makes it possible to produce surface-modified zinc oxide particles having a small color difference ΔE.

It is common to hydrolyze the surface treatment agent in advance, but the fact that the color difference ΔE of surface-modified zinc oxide particles surface-treated with a hydrolyzed surface treatment agent is significantly decreased is a nonobvious effect exceeding the scope of expectation by those skilled in the art.

A hydrolysate may be prepared by holding a liquid mixture of a surface treatment agent and water at a constant temperature for a predetermined time. This makes it possible for the hydrolysis of the surface treatment agent to be further accelerated.

In this treatment, the temperature of the liquid mixture is not particularly limited and can be changed as appropriate depending on the kind of the surface treatment agent, but is, for example, preferably 5°C or higher and 65°C or lower and more preferably 30°C or higher and 60°C or lower. For example, the temperature may be 10°C or higher and 50°C or lower, 15°C or higher and 40°C or lower, 20°C or higher and 35°C or lower, or the like as necessary.

In addition, the holding time is not particularly limited and is, for example, preferably 10 minutes or longer and 180 minutes or shorter and more preferably 30 minutes or longer and 120 minutes or shorter. During the above-described holding of the liquid mixture, the liquid mixture may be stirred as appropriate.

The surface treatment agent that is used in the first step can be the same as the above-described one and thus will not be described again.

Water is not particularly limited as long as the water is water that is commonly used in cosmetic preparations, and pure water, ion-exchanged water, distilled water, purified water, ultrapure water, natural water, and the like can be used.

In the present step, solvents other than water may not be contained, but it is preferable to mix a solvent other than water in order to control the hydrolysis reaction of the surface treatment agent. The solvent is not particularly limited as long as the solvent does not excessively control the hydrolysis of the surface treatment agent, and, for example, an alcoholic solvent can be mixed in.

Examples of the alcoholic solvent include branched or linear alcohol compounds having 1 to 4 carbon atoms, and these can be used singly or two or more thereof may be used in combination. In addition, the alcohol compound that is exemplified as the alcoholic solvents may be any of primary, secondary and tertiary alcohols. In addition, the alcohol compound that is exemplified as the alcoholic solvent may be any of monohydric, divalent, and trihydric alcohols. More specifically, examples of the alcoholic solvent include methanol, ethanol, 1-propanol, isopropyl alcohol, 1-butyl alcohol, 2-butanol, isobutyl alcohol, tert-butyl alcohol, methanediol, 1,2-ethanediol, 1,2-propanediol, 1,3-propanediol, 1,2-butanediol, 1,3-butanediol, 1,4-butanediol, 2,3-butanediol, 2-butene-1,4-diol, 1,4-butynediol, glycerin, diethylene glycol, 3-methoxy-1,2-propanediol, and the like.

Among these alcoholic solvents, ethanol and isopropyl alcohol are preferable from the viewpoint of the easy control of the hydrolysis reaction and a small influence thereof even when mixed into cosmetic preparations.

In the present step, a catalyst may be mixed in order to accelerate the hydrolysis reaction of the surface treatment agent.

The catalyst may be an acid or may be a base. The acid catalyzes the hydrolysis reaction of the surface treatment agent in a dispersion liquid. On the other hand, the base catalyzes the condensation reaction of the hydrolyzed surface treatment agent with silanol groups on the particle surfaces.

Examples of the acid include inorganic acids such as hydrochloric acid, hydrobromic acid, hydroiodic acid, sulfuric acid, nitric acid, boric acid, and phosphoric acid and organic acids such as acetic acid, citric acid and formic acid. These acids may be used singly or two or more acids may be used in combination.

Examples of the base include sodium hydroxide, potassium hydroxide, barium hydroxide, calcium hydroxide, ammonia, amines, and the like. These bases may be used singly or two or more bases may be used in combination.

The content of the surface treatment agent in the liquid mixture can be arbitrarily selected.

The content of the surface treatment agent in the liquid mixture is not particularly limited, but is, for example, preferably 20% by mass or more and 70% by mass or less and more preferably 30% by mass or more and 60% by mass or less. The content may be, for example, 25% by mass or more and 45% by mass or less, 35% by mass or more and 55% by mass or less, 45% by mass or more and 65% by mass or less, or the like.

The content of water in the liquid mixture is not particularly limited, but is, for example, preferably 1% by mass or more and 20% by mass or less, more preferably 3% by mass or more and 15% by mass or less, and still more preferably 5% by mass or more and 10% by mass or less.

The content of the solvent in the liquid mixture is not particularly limited, but is, for example, preferably 20% by mass or more and 70% by mass or less and more preferably 30% by mass or more and 60% by mass or less. This makes it possible to sufficiently increase the content of the surface treatment agent in the hydrolysate and makes it possible to contain a sufficient amount of water in the liquid mixture. As a result, it is possible to make the hydrolysis reaction of the surface treatment agent proceed efficiently.

The content of the catalyst in the liquid mixture is not particularly limited, but is, for example, preferably 10 ppm or more and 1000 ppm or less and more preferably 20 ppm or more and 800 ppm or less. This makes it possible for the hydrolysis reaction of the surface treatment agent to be sufficiently accelerated.

### "Second step of preparing first mixture (first surface treatment step)"

The present step is a step of producing a mixture by mixing the hydrolysate and the zinc oxide particles to perform the surface treatment of the zinc oxide particles with the surface treatment agent in a dry manner.

The zinc oxide particles are the same as the above-described one and thus will not be described again.

A method for mixing the hydrolysate and the zinc oxide particles is not particularly limited, and the hydrolysate and the zinc oxide particles may be mixed with a stirring intensity high enough to attach the surface treatment agent to the surfaces of the zinc oxide particles. For example, the zinc oxide particles and the hydrolysate are injected into a mixer such as a Henschel mixer or a super mixer and strongly stirred at a high speed for a certain time. From the viewpoint of accelerating the surface treatment reaction, it is preferable to perform a heating treatment at an arbitrarily selected temperature, for example, a temperature of 70°C to 200°C while continuously stirring the zinc oxide particles and the hydrolysate. The heating treatment may be performed at, for example, 70°C to 90°C, 90°C to 120°C, 120°C to 150°C, 150°C to 200°C, or the like as necessary. The mixing time can be arbitrarily selected. The mixing time can be, for example, 0.1 to 10 hours and more preferably 0.3 to 7 hours, but is not limited to these examples.

The mixing ratio between the hydrolysate and the zinc oxide particles is not particularly limited, but it is preferable to mix the hydrolysate and the zinc oxide particles so that the surface treatment agent becomes 1% by mass or more and 20% by mass or less in the total mass of the zinc oxide particles and the surface treatment agent after a third step to be described below. The mixing ratio is more preferably 5% by mass or more and 18% by mass or less and still more preferably 8% by mass or more and 16% by mass or less. An obtained mixture may be in a somewhat dried state or in a non-dried state as necessary.

### "Third step of preparing second mixture (second surface treatment step)"

The present step may or may not be performed after the second step.

The third step may be performed on the mixture obtained in the second step as it is, but the present step may be performed after the first mixture obtained in the second step is dried at an arbitrarily selected temperature, for example, 60°C to 100°C to remove moisture. The drying time can be arbitrarily selected.

The present step is a step in which the first mixture obtained in the second step or the first mixture from which moisture has been removed after being obtained in the second step and the hydrolysate prepared in the first step are mixed again to produce a second mixture, thereby performing the surface treatment of the zinc oxide particles with the surface treatment agent one more time.

The surface treatment performed twice makes it easy for the surface treatment agent to be more uniformly attached to the particle surfaces. The number of times of the surface treatment step is not limited to one or two, the surface treatment step may be performed three times to form a third mixture, four times to form a fourth mixture, or five or more times. The step of mixing the hydrolysate obtained in the first step and the mixture obtained in the step can be repeated once or more. That is, the surface treatment step can be repeated once or more times until intended particles are obtained.

As a mixing method, the first mixture and the hydrolysate may be mixed with a stirring intensity high enough to attach the surface treatment agent to the surfaces of the zinc oxide particles as in the second step. A heating treatment may be performed at an arbitrarily selected temperature for an arbitrarily selected time, for example, at a temperature of 70°C to 200°C for 0.1 to 10 hours while continuously stirring the first mixture and the hydrolysate as in the second step.

The mixing ratio between the hydrolysate and the zinc oxide particles is not particularly limited, but it is preferable to mix the hydrolysate and the zinc oxide particles so that the surface treatment agent becomes 1% by mass or more and 20% by mass or less in the total mass of the zinc oxide particles and the surface treatment agent after the second step. The mixing ratio is more preferably 5% by mass or more and 18% by mass or less and still more preferably 8% by mass or more and 16% by mass or less.

The mass ratio between the surface treatment agent mixed in the second step and the surface treatment agent mixed in the third step is not particularly limited, but is preferably 0.5:1 to 3:1. The mass ratio may be 0.6:1 to 2:1, 0.7:1 to 1.5:1, or the like as necessary.

Since the second and subsequent surface treatment steps are performed to suppress the remaining of hydrolyzable reactive groups, it is preferable to reduce the mixing ratio of the surface treatment agent and accelerate the surface treatment by heating or the like.

### "Drying step"

The present step is performed after the surface treatment step to remove water or the solvent remaining in the mixture such as the first mixture or the second mixture.

In the present step, the first mixture or the second mixture may be heated with a drying device until water or the solvent is removed.

The drying device is not particularly limited, and examples thereof include a box-like dryer, a vacuum dryer, a vibration dryer, a fluidized bed dryer, a band dryer, an evaporator, a NAUTA mixer, a Henschel mixer, a RIBOCONE, a paddle dryer, a spray dryer, a slurry dryer, a flash dryer, a rotary dryer, and the like.

The drying temperature and the drying time are not particularly limited, and the mixture may be dried, for example, at 60°C to 130°C for 10 minutes to 24 hours.

### "Cracking step"

The present step may not be performed, may be performed before the drying step, and/or may be performed after the drying step. When the present step is performed, it is possible to loosen the agglomeration of the surface-modified zinc oxide particles or to suppress a rough feel when the surface-modified zinc oxide particles are blended into cosmetic preparations. That is, the cracking step makes it possible to improve usability when the surface-modified zinc oxide particles are used in cosmetic preparations.

In the cracking step, a cracking treatment can be performed using a cracking device. The cracking device can be arbitrarily selected, and examples thereof include an atomizer, a hammer mill, a jet mill, an impeller mill, a pin mill, and the like.

The above-described steps make it possible to produce the surface-modified zinc oxide particles of the present embodiment in a dry manner.

### (Production method in wet manner)

Next, a method for producing the surface-modified zinc oxide particles of the present embodiment by a wet treatment will be described.

### "First step of preparing hydrolysate"

The present step is the same as the above-described dry treatment and will not be described again.

### "Step of preparing first mixture (first surface treatment step)"

The present step is a step of producing a first mixture by mixing the hydrolysate, the zinc oxide particles, and a solvent to perform the surface treatment of the zinc oxide particles with the surface treatment agent in a wet manner.

Mixing is preferably performed using a disperser from the viewpoint of uniformly treating the surfaces of the zinc oxide particles with the surface treatment agent. Therefore, mixing using a disperser will be described in detail.

The zinc oxide particles are the same as the above-described one and thus will not be described again.

In the present step, the hydrolysate, a solvent, and zinc oxide particles are put into a disperser to produce a liquid mixture. These materials may be put in simultaneously or sequentially. The order of putting in these materials is not particularly limited. After being put into the disperser, these materials may be left to stand as they are and not stirred, but may be stirred for a long time or may be stirred briefly for a short time. In addition, before being put into the disperser, these materials may be mixed in advance and then put into the disperser.

### "Solvent"

The solvent is not particularly limited as long as the solvent is miscible with the surface treatment agent. Examples of the solvent include alcohols such as methanol, ethanol, n-propanol, isopropanol, and n-butanol, esters such as ethyl acetate and butyl acetate, n-hexane, toluene, xylene, and the like. It is also possible to use the solvents exemplified in the "surface treatment in a dry manner" section. Among these solvents, alcohols are preferable since alcohols are miscible with water, and, among alcohols, ethanol is particularly preferable. These solvents may be used singly or two or more solvents may be used in combination.

The content of the solvent in the liquid mixture can be arbitrarily selected, but is preferably 40% by mass or more in order to suppress agglomeration of the zinc oxide particles. The upper limit value of the content of the solvent is not particularly limited, but is preferably 95% by mass or less from the viewpoint of the production efficiency.

The content of the zinc oxide particles in the liquid mixture can be arbitrarily selected, but is preferably 1% by mass or more and 55% by mass or less and more preferably 10% by mass or more and 50% by mass or less from the viewpoint of satisfying both the suppression of agglomeration of the zinc oxide particles and the production efficiency. The content may be 15% by mass or more and 45% by mass or less, 20% by mass or more and 40% by mass or less, 25% by mass or more and 35% by mass or less, or the like.

The mixing ratio between the hydrolysate and the zinc oxide particles is not particularly limited, but it is preferable to mix the hydrolysate and the zinc oxide particles so that the surface treatment agent becomes 1% by mass or more and 20% by mass or less in the total mass of the zinc oxide particles and the surface treatment agent after a third step to be described below. The mixing ratio is more preferably 5% by mass or more and 18% by mass or less and still more preferably 8% by mass or more and 16% by mass or less.

### "Disperser"

The disperser is not particularly limited as long as the disperser is capable of imparting dispersion energy to the liquid mixture to an extent that the surface treatment can be performed while loosening the agglomeration of the zinc oxide particles.

Examples of such a disperser include a colloid mill, a roll mill, an ultrasonic disperser, a high-pressure homogenizer, an ultimizer, a rotary mill, a planetary mill, a bead mill, a sand mill, and the like. As dispersion media that are used in dispersion devices that require dispersion media, it is possible to use, for example, granules having predetermined hardness of zirconia, glass, alumina, titania, silicon nitride, or the like.

In the present step, the liquid mixture is dispersed using the disperser with a predetermined amount of energy or more to obtain surface-modified zinc oxide particles.

The energy that is imparted to the liquid mixture may be appropriately adjusted according to the size or kind of the disperser. Therefore, the conditions of the dispersion may be selected as appropriate. For example, in a case where the dispersion treatment is performed with a mill, specifically, a bead mill using a container having a capacity of about 1 L, it is preferable to perform the dispersion treatment at a rotation speed of 500 rpm or faster for 1 hour or longer and 10 hours or shorter. Here, the conditions can be selected as necessary and are not limited only to the above conditions.

In addition, in a case where the dispersion treatment is performed with a mill, specifically, a bead mill using a container having a capacity of about 1 L, the dispersion energy that is imparted to the liquid mixture is, for example, preferably 100 W-h/kg or higher and 600 W-h/kg or lower. Here, the conditions can be selected as necessary and are not limited only to the above conditions.

In addition, since the cracking force of the bead mill depends on the centrifugal force of a disc or beads on the pin outer periphery in the mill, when the product (impulse) of the centrifugal force and the dispersion time is calculated in a case where the total weight of the beads in the mill is regarded as the bead weight, the dispersion treatment is preferably performed such that the product becomes 0.5 × 10⁶ N.s or higher and 100 × 10⁶ N.s or lower.

In addition, the dispersion treatment may be performed until b* of the surface-modified zinc oxide particles becomes 4.0 or higher and 18 or lower. In this case, the degree of progress of the dispersion treatment may be confirmed by extracting a small amount of the zinc oxide particles during the dispersion treatment and measuring b* of the zinc oxide particles using a spectral colorimeter.

The temperature in the step of dispersing the liquid mixture with the disperser is not particularly limited, but is, for example, preferably 20°C or higher and 45°C or lower.

### "Third step of preparing second mixture (second surface treatment step)"

The present step may or may not be performed after the second step.

The present step is a step of mixing the first mixture obtained in the second step and the hydrolysate again to produce a second mixture, thereby performing the surface treatment of the zinc oxide particles with the surface treatment agent one more time.

The surface treatment performed twice makes it easy for the surface treatment agent to be more uniformly attached to the particle surfaces. The surface treatment step may be performed three times, four times, or five or more times. That is, the surface treatment step can be repeated once or more times until intended particles are obtained.

The mixing ratio between the hydrolysate and the zinc oxide particles is not particularly limited, but it is preferable to mix the hydrolysate and the zinc oxide particles so that the surface treatment agent becomes 1% by mass or more and 20% by mass or less in the total mass of the zinc oxide particles and the surface treatment agent after the second step. The mixing ratio is more preferably 5% by mass or more and 18% by mass or less and still more preferably 8% by mass or more and 16% by mass or less.

The mass ratio between the surface treatment agent mixed in the second step and the surface treatment agent mixed in the third step is not particularly limited, but is preferably 0.5:1 to 3:1. The mass ratio may be 0.6:1 to 2:1, 0.7:1 to 1.5:1, or the like as necessary.

Since the second and subsequent surface treatment steps are performed to suppress the remaining of hydrolyzable reactive groups, it is preferable to reduce the mixing ratio of the surface treatment agent to be smaller in the first surface treatment step and accelerate the surface treatment by heating or the like.

Since the present step is a step performed to suppress the remaining of hydrolyzable reactive groups, the first mixture and the hydrolysate are preferably mixed while being heated.

That is, in the case of performing the surface treatment in a wet manner, the surface treatment agent is uniformly attached to the surfaces of the zinc oxide particles in the second step, and the surface treatment is further accelerated in the third step.

The heating temperature is not particularly limited as long as the surface treatment is accelerated at the heating temperature and is, for example, preferably 40°C or higher and 150°C or lower. The heating temperature may be 40°C or higher and 80°C or lower, 60°C to 100°C, or the like as necessary.

### "Drying step"

The present step is performed to remove water and the solvent remaining in the second mixture. In the present step, it is preferable to dry the second mixture using a drying device. A treatment that shortens the drying time, such as solid-liquid separation, may be performed before drying. The drying device is not particularly limited, and examples thereof include a box-like dryer, a vacuum dryer, a vibration dryer, a fluidized bed dryer, a band dryer, an evaporator, a NAUTA mixer, a Henschel mixer, a RIBOCONE, a paddle dryer, a spray dryer, a slurry dryer, a flash dryer, a rotary dryer, and the like.

The drying temperature is not particularly limited as long as the solvent can be removed, but is preferably, for example, 50°C or higher and 200°C or lower. The drying temperature may be 60°C or higher and 150°C or lower, 70°C or higher and 120°C or lower, or the like.

### "Cracking step"

The dried surface-modified zinc oxide particles may be cracked using a cracking device. The cracking device can be arbitrarily selected, and examples thereof include an atomizer, a hammer mill, a jet mill, an impeller mill, a pin mill, and the like. The cracking step makes it possible to suppress the rough feel of the surface-modified zinc oxide particles when, for example, the surface-modified zinc oxide particles are blended with cosmetic preparations. That is, the cracking step makes it possible to improve usability when the surface-modified zinc oxide particles are used in cosmetic preparations.

The above-described steps make it possible to produce the surface-modified zinc oxide particles of the present embodiment in a wet manner.

### [Second embodiment]

A second embodiment may use and/or have the characteristics or conditions in the first embodiment in the same manner unless particularly otherwise described.

### [Surface-modified zinc oxide particles]

Surface-modified zinc oxide particles of the present embodiment are surface-modified zinc oxide particles in which the particle surfaces of zinc oxide particles are treated with a surface treatment agent, in which the color difference ΔE before and after irradiation with simulated sunlight is 4.0 or lower, and the total light transmittance at a wavelength of 360 nm after exposure to an environment of 85°C and 95 %RH for 48 hours is 40% or lower.

The color difference ΔE is preferably 3.5 or lower, more preferably 3.0 or lower, still more preferably 2.5 or lower, and particularly preferably 2.0 or lower.

The lower limit value of the color difference ΔE is 0, and may be 0.01, may be 0.05, or may be 0.1.

The surface-modified zinc oxide particles of the present embodiment have a color difference ΔE of 4.0 or lower and thus have excellent ultraviolet shielding properties.

The color difference ΔE before and after irradiation with simulated sunlight in the present embodiment means a color difference measured by the same measurement method as in the first embodiment.

As described in the first embodiment as well, when the mixture containing the surface-modified zinc oxide particles and t-butyl methoxydibenzoylmethane is exposed to simulated sunlight, if the surface treatment is not sufficient, t-butyl methoxydibenzoylmethane is altered due to the photocatalytic activity of the zinc oxide particles or the elution of zinc ions, and the mixture discolors.

The present inventors and the like found that, if a surface treatment is performed on the zinc oxide particles so that the color difference ΔE becomes 4.0 or lower, the ultraviolet shielding properties of the surface-modified zinc oxide particles improve and completed the present invention. In addition, the present inventors and the like found that surface-modified zinc oxide particles having superior transparency and a color difference ΔE of 4.0 or lower can be obtained by performing the surface treatment with the hydrolyzable surface modifier twice or more by a wet method using a hydrolysate that has been hydrolyzed in advance. Furthermore, it was found that the temporal stability is improved by surface-treating the zinc oxide particles with a predetermined amount of the surface treatment agent.

As described in the first embodiment as well, in the present embodiment, the reason for the ultraviolet shielding properties of the surface-modified zinc oxide particles to be improved when the color difference ΔE becomes 4.0 or lower is not clear. The reason for the color difference ΔE to become small is presumed that the photocatalytic activity or the elution of zinc ions is suppressed. However, there is no method for directly measuring how the hydrolyzable surface modifier modifies the surfaces of the zinc oxide particles. Therefore, it is not clear that how the previously-hydrolyzed surface modifier surface-modifies the surfaces of the zinc oxide particles leads to a decrease in the color difference ΔE. The reason is also presumed that the surface modifier is uniformly and densely present on the surfaces of the zinc oxide particles, which is not enough to describe everything.

In the present embodiment, the effect of the use of the previously-hydrolyzed surface treatment agent enabling the improvement in the ultraviolet shielding properties of the surface-modified zinc oxide particles is presumed to develop due to a number of factors intricately intertwined. Specifically, the effect is presumed to develop due to a number of factors intricately intertwined such as the shapes, specific surface area, and particle size distribution of the zinc oxide particles, the degree of hydrolysis of the hydrolyzable surface modifier, the attachment condition of the hydrolyzable surface modifier to the zinc oxide particles, and the attachment rate of the hydrolyzable surface modifier to the zinc oxide particles. Therefore, it is considered that it is almost impossible to directly specify the characteristics of the surface-modified zinc oxide particles of the present embodiment with the state of the surfaces of the zinc oxide particles surface-modified with the hydrolyzable surface treatment agent that has been hydrolyzed in advance.

As a result of various studies, the present inventors and the like focused on the color difference ΔE and found that the ultraviolet shielding properties are improved and the temporal stability are also improved by surface-treating the zinc oxide particles with a predetermined amount of a previously-hydrolyzed hydrolyzable surface treatment agent so that the color difference ΔE becomes 4.0 or lower.

In the surface-modified zinc oxide particles of the present embodiment, the total light transmittance at a wavelength of 360 nm measured by the following measurement method is preferably 40% or lower, more preferably 38% or lower, still more preferably 37% or lower, and particularly preferably 35% or lower.

A cosmetic preparation having particularly excellent ultraviolet shielding properties can be obtained by blending in surface-modified zinc oxide particles having a total light transmittance at a wavelength of 360 nm, that is, a sum of the linear transmittance and the diffuse transmittance, of 40% or lower.

The lower limit of the total light transmittance at a wavelength of 360 nm may be 0%, may be 1%, may be 10%, or may be 20%.

In the surface-modified zinc oxide particles of the present embodiment, the linear transmittance at a wavelength of 550 nm measured by the following measurement method is preferably 85% or higher, more preferably 90% or higher, and still more preferably 92% or higher.

A cosmetic preparation having excellent transparency can be obtained by blending in surface-modified zinc oxide particles having a linear transmittance at a wavelength of 550 nm of 85% or higher.

The upper limit of the linear transmittance at a wavelength of 550 nm may be 100%, may be 99%, or may be 98%.

In the present embodiment, the total light transmittance at a wavelength of 360 nm and the linear transmittance at a wavelength of 550 nm means values measured by the same measurement method as in the first embodiment.

In the surface-modified zinc oxide particles of the present embodiment, the particle diameter (d90) when the cumulative volume percentage of the particle size distribution measured by the same measurement method as in the first embodiment is 90% is preferably 0.8 µm or lower, more preferably 0.6 µm or lower, still more preferably 0.4 µm or lower, and particularly preferably 0.3 µm or lower.

A cosmetic preparation having excellent transparency and ultraviolet shielding properties can be obtained by blending surface-modified zinc oxide particles having d90 of the above-described upper limit value or lower into the cosmetic preparation.

The lower limit of d90 may be 0.05 µm or may be 0.1 µm.

In the surface-modified zinc oxide particles of the present embodiment, the total light transmittance at a wavelength of 360 nm after the accelerated test where the surface-modified zinc oxide particles are exposed to an environment of 85°C and 95 %RH for 48 hours is 40% or lower. The total light transmittance may be 38% or lower, 35% or lower, or 30% or lower as necessary. A cosmetic preparation having particularly excellent temporal stability can be obtained by blending surface-modified zinc oxide particles having a total light transmittance at a wavelength of 360 nm after the accelerated test of 40% or lower into the cosmetic preparation.

The lower limit of the total light transmittance at a wavelength of 360 nm after the accelerated test may be 0%, may be 1%, may be 10%, or may be 20%.

The total light transmittance at a wavelength of 360 nm of the surface-modified zinc oxide particles in a state where the accelerated test is not performed may have the same value as the value described in the first embodiment.

In the surface-modified zinc oxide particles of the present embodiment, the linear transmittance at a wavelength of 550 nm after the accelerated test where the surface-modified zinc oxide particles are exposed to an environment of 85°C and 95 %RH for 48 hours is preferably 90% or higher. The linear transmittance may be, for example, 93% or higher or may be 95% or higher. A cosmetic preparation having excellent temporal stability can be obtained by blending surface-modified zinc oxide particles having a linear transmittance at a wavelength of 550 nm after the accelerated test of 90% or higher into the cosmetic preparation.

The upper limit of the linear transmittance at a wavelength of 550 nm after the accelerated test may be 100%, may be 99%, or may be 98%.

In the surface-modified zinc oxide particles of the present embodiment, d90 after the accelerated test where the surface-modified zinc oxide particles are exposed to an environment of 85°C and 95 %RH for 48 hours is preferably 0.3 um or less. For example, d90 may be 0.25 µm or less or 0.20 µm or less. A cosmetic preparation having excellent temporal stability can be obtained by blending surface-modified zinc oxide particles having d90 after the accelerated test of 0.3 µm or less into the cosmetic preparation.

The lower limit of d90 may be 0.05 µm or may be 0.1 µm.

The specific surface area of the surface-modified zinc oxide particles of the present embodiment can be arbitrarily selected, but is preferably 1.5 m²/g or more, more preferably 2.5 m²/g or more, and still more preferably 4 m²/g or more. In addition, the specific surface area of the surface-modified zinc oxide particles may be, for example, 65 m²/g or less and is preferably 55 m²/g or less, and more preferably 50 m²/g or less. The specific surface area of the surface-modified zinc oxide particles may be 45 m²/g or less, may be 40 m²/g or less, or may be 20 m²/g or less as necessary. The upper limit value and lower limit value of the specific surface area of the surface-modified zinc oxide particles can be arbitrarily combined together.

When the specific surface area of the surface-modified zinc oxide particles is 1.5 m²/g or more and 65 m²/g or less, transparency and ultraviolet shielding properties are excellent in a case where the surface-modified zinc oxide particles are blended into a cosmetic preparation.

In a case where there is a desire to enhance the transparency in the case of blending the surface-modified zinc oxide particles into a cosmetic preparation, the specific surface area of the surface-modified zinc oxide particles is preferably 8 m²/g or more, more preferably 15 m²/g or more, and still more preferably 20 m²/g or more.

For example, the specific surface area of the surface-modified zinc oxide particles is preferably 20 m²/g or more and 65 m²/g or less, more preferably 20 m²/g or more and 60 m²/g or less, and still more preferably 20 m²/g or more and 55 m²/g or less. The specific surface area may be 20.0 m²/g or more and 30.0 m²/g or less, 20.0 m²/g or more and 38.0 m²/g or less, or 20.0 m²/g or more and 50.0 m²/g or less as necessary. In a case where the specific surface area of the surface-modified zinc oxide particles is the lower limit value or higher, it is possible to obtain a cosmetic preparation having excellent transparency when the surface-modified zinc oxide particles are blended into the cosmetic preparation. On the other hand, when the specific surface area of the surface-modified zinc oxide particles is the upper limit value or lower, since the surface energy of the particles is not too large, it is possible to blend the surface-modified zinc oxide particles into a cosmetic preparation with a small amount of energy.

Incidentally, in a case where there is a desire to enhance the ultraviolet shielding properties in the UVA region in the case of blending the surface-modified zinc oxide particles into a cosmetic preparation, the specific surface area of the surface-modified zinc oxide particles is preferably less than 20 m²/g, more preferably 15 m²/g or less, and still more preferably less than 8 m²/g.

For example, the specific surface area of the surface-modified zinc oxide particles is preferably 1.5 m²/g or more and less than 20 m²/g, more preferably 1.5 m²/g or more and 15 m²/g or less, and still more preferably 1.5 m²/g or more and less than 8 m²/g. In a case where the specific surface area of the surface-modified zinc oxide particles is the lower limit value or higher, it is possible to obtain a cosmetic preparation having transparency when the surface-modified zinc oxide particles are blended into the cosmetic preparation. On the other hand, when the specific surface area of the surface-modified zinc oxide particles is less than the upper limit value, since the surface energy of the particles is not too large, it is possible to blend the surface-modified zinc oxide particles into a cosmetic preparation with a small amount of energy and to obtain a cosmetic preparation having excellent ultraviolet shielding properties in the UVA region.

The specific surface area (unit: m²/g) of the surface-modified zinc oxide particles in the present embodiment is the BET specific surface area obtained by the BET method in the same manner as in the first embodiment.

Examples of a method for measuring the specific surface area of the surface-modified zinc oxide particles include a BET method in which a full automatic specific surface area-measuring instrument (for example, trade name: Macsorb HM Model-1201, manufactured by Mountech Co., Ltd.) is used.

In the present embodiment, "being treated with a surface treatment agent" refers to the fact that the surface treatment agent comes into contact with or bonds to the zinc oxide particles by an interaction therebetween. As the contact, for example, physical adsorption is exemplified. In addition, as the bond, for example, an ionic bond, a hydrogen bond, a covalent bond, and the like are exemplified.

The amount of the surface treatment agent in the surface-modified zinc oxide particles may be appropriately adjusted in accordance with the specific surface area of the zinc oxide particles and the degree of hydrophobilization of a cosmetic preparation into which the surface-modified zinc oxide particles are to be blended before use. For example, the amount of the surface treatment agent in the surface-modified zinc oxide particles is preferably 1% by mass or more and 20% by mass or less, more preferably 5% by mass or more and 18% by mass or less, and still more preferably 8% by mass or more and 16% by mass or less of the total mass of the zinc oxide particles and the surface treatment agent. When the amount of the surface treatment agent is the lower limit value or more, it is possible to blend hydrophilic zinc oxide particles into an oily cosmetic preparation. In addition, when the amount of the surface treatment agent is the upper limit value or less, the ultraviolet shielding properties of the zinc oxide particles when blended into a cosmetic preparation are sufficiently exhibited.

From the viewpoint of decreasing the color difference ΔE and improving the temporal stability, the content of the surface treatment agent is preferably 4% by mass or more and 20% by mass or less, more preferably 6% by mass or more and 18% by mass or less, and still more preferably 8% by mass or more and 16% by mass or less of the total of the zinc oxide particles and the surface treatment agent.

In a case where the surface treatment agent is a silane coupling agent, the content of the surface treatment agent in the surface-modified zinc oxide particles can be calculated by, for example, quantitatively analyzing the amount of Si in the surface-modified zinc oxide particles with an inductively coupled plasma atomic emission spectrometer.

### "Zinc oxide particles"

As the zinc oxide particles (before the surface treatment) in the present embodiment, the same zinc oxide particles as in the first embodiment can be used.

### "Hydrolyzable surface treatment agent"

The hydrolyzable surface treatment agent of the present embodiment is not particularly limited as long as the surface treatment agent is hydrolyzable and can be used in cosmetic preparations, and the same hydrolyzable surface treatment agent as in the first embodiment can be used.

In the surface-modified zinc oxide particles of the present embodiment, the zinc oxide particles may be surface-treated using, in addition to the silane coupling agent, the surface treatment agent that is used for cosmetic preparations and is not a silane coupling agent as long as the characteristics of the surface-modified zinc oxide particles are not impaired.

According to the surface-modified zinc oxide particles of the present embodiment, in the surface-modified zinc oxide particles, the particle surfaces of zinc oxide particles are treated with the hydrolyzable surface treatment agent, the color difference ΔE before and after irradiation with simulated sunlight is 4.0 or lower, and the total light transmittance at a wavelength of 360 nm after exposure to an environment of 85°C and 95 %RH for 48 hours is 40% or lower, and thus the ultraviolet shielding properties are excellent, and the temporal stability is excellent.

### "Method for producing surface-modified zinc oxide particles"

A method for producing surface-modified zinc oxide particles of the present embodiment is a method for producing the above-described surface-modified zinc oxide particles, having a first step of mixing a surface treatment agent and water to prepare a hydrolysate, a second step of mixing the hydrolysate and zinc oxide particles to prepare a first mixture, a third step of mixing the first mixture obtained in the second step and the hydrolysate obtained in the first step to prepare a second mixture containing the first mixture and the hydrolysate, and a step of drying the second mixture obtained in the third step.

The drying step may be performed after the second step without performing the third step.

The surface treatment of the present embodiment is performed not in a dry manner but in a wet manner.

### (Production method in wet manner)

### "First step of preparing hydrolysate"

In the present step, a surface treatment agent and water are mixed to prepare a hydrolysate. The use of a hydrolysate in which at least a part of the surface treatment agent has been hydrolyzed in advance as described above makes it possible to produce surface-modified zinc oxide particles having a small color difference ΔE.

It is common to hydrolyze the surface treatment agent in advance, but the fact that the color difference ΔE of surface-modified zinc oxide particles surface-treated with a hydrolyzed surface treatment agent is significantly decreased is a nonobvious effect exceeding the scope of expectation by those skilled in the art.

A hydrolysate may be prepared by holding a liquid mixture of a surface treatment agent and water at a constant temperature for a predetermined time. This makes it possible for the hydrolysis of the surface treatment agent to be further accelerated.

In this treatment, the temperature of the liquid mixture is not particularly limited and can be changed as appropriate depending on the kind of the surface treatment agent, but is, for example, preferably 5°C or higher and 65°C or lower and more preferably 30°C or higher and 60°C or lower. For example, the temperature may be 10°C or higher and 50°C or lower, 15°C or higher and 40°C or lower, 20°C or higher and 35°C or lower, or the like as necessary.

In addition, the holding time is not particularly limited and is, for example, preferably 10 minutes or longer and 180 minutes or shorter and more preferably 30 minutes or longer and 120 minutes or shorter. During the above-described holding of the liquid mixture, the liquid mixture may be stirred as appropriate.

The surface treatment agent that is used in the first step can be the same as the above-described one and thus will not be described again.

Water is not particularly limited as long as the water is water that is commonly used in cosmetic preparations, and pure water, ion-exchanged water, distilled water, purified water, ultrapure water, natural water, and the like can be used.

In the present step, it is preferable to mix a solvent other than water in order to control the hydrolysis reaction of the surface treatment agent. The solvent is not particularly limited as long as the solvent does not excessively control the hydrolysis of the surface treatment agent, and, for example, an alcoholic solvent can be mixed in.

As the alcoholic solvent, the alcoholic solvent, kind, or conditions described in the first step of the first embodiment can also be preferably used in the present embodiment.

In the present step, a catalyst may be mixed in order to accelerate the hydrolysis reaction of the surface treatment agent.

As the catalyst, the catalyst or conditions described in the first step of the first embodiment can also be preferably used in the present embodiment.

As an acid, the acid or conditions described in the first step of the first embodiment can also be preferably used in the present embodiment.

The content of the surface treatment agent in the liquid mixture is not particularly limited, but is, for example, preferably 20% by mass or more and 70% by mass or less and more preferably 30% by mass or more and 60% by mass or less. The content may be, for example, 25% by mass or more and 45% by mass or less, 35% by mass or more and 55% by mass or less, 45% by mass or more and 65% by mass or less, or the like.

The content of water in the liquid mixture is not particularly limited, but is, for example, preferably 1% by mass or more and 20% by mass or less, more preferably 3% by mass or more and 15% by mass or less, and still more preferably 5% by mass or more and 10% by mass or less.

The content of the solvent in the liquid mixture is not particularly limited, but is, for example, preferably 20% by mass or more and 70% by mass or less and more preferably 30% by mass or more and 60% by mass or less. This makes it possible to sufficiently increase the content of the surface treatment agent in the hydrolysate and makes it possible to contain a sufficient amount of water in the liquid mixture. As a result, it is possible to make the hydrolysis reaction of the surface treatment agent proceed efficiently.

The content of the catalyst in the liquid mixture is not particularly limited, but is, for example, preferably 10 ppm or more and 1000 ppm or less and more preferably 20 ppm or more and 800 ppm or less. This makes it possible for the hydrolysis reaction of the surface treatment agent to be sufficiently accelerated.

### "Step of preparing first mixture (first surface treatment step)"

The present step is a step of producing a first mixture by mixing the hydrolysate and the zinc oxide particles to perform the surface treatment of the zinc oxide particles with the surface treatment agent in a wet manner.

The present step may be a step of producing a mixture by mixing the hydrolysate, the zinc oxide particles, and a solvent to perform the surface treatment of the zinc oxide particles with the surface treatment agent in a wet manner.

That is, a solvent may be mixed in a case where the amount of the hydrolysate is small and the treatment does not become a wet treatment. The wet treatment in the present embodiment means that the surface treatment is performed in a state where 40% by mass or more in total of the solvent, including the solvent contained in the hydrolysate, is present in the mixture.

Mixing is preferably performed using a disperser from the viewpoint of uniformly treating the surfaces of the zinc oxide particles with the surface treatment agent. Therefore, mixing using a disperser will be described in detail.

The zinc oxide particles are the same as the above-described one and thus will not be described again.

In the present step, the hydrolysate, a solvent, and zinc oxide particles are put into a disperser to produce a liquid mixture. These materials may be put in simultaneously or sequentially. The order of putting in these materials is not particularly limited. After being put into the disperser, the materials may not be stirred or may be simply stirred. In addition, before being put into the disperser, these materials may be mixed in advance and then put into the disperser.

### "Solvent"

The solvent is not particularly limited as long as the solvent is miscible with the surface treatment agent. The solvent or description described in the wet treatment of the first embodiment can also be preferably used in the present step of the present embodiment.

The content of the solvent in the liquid mixture can be arbitrarily selected, but is preferably 40% by mass or more in order to suppress agglomeration of the zinc oxide particles. The upper limit value of the content of the solvent is not particularly limited, but is preferably 95% by mass or less from the viewpoint of the production efficiency.

The content of the zinc oxide particles in the liquid mixture can be arbitrarily selected. The content or description of the zinc oxide particles described in the wet treatment of the first embodiment can also be preferably used in the present step of the present embodiment.

The mixing ratio between the hydrolysate and the zinc oxide particles is not particularly limited, but it is preferable to mix the hydrolysate and the zinc oxide particles so that the surface treatment agent becomes 1% by mass or more and 20% by mass or less in the total mass of the zinc oxide particles and the surface treatment agent after a third step to be described below. The mixing ratio is more preferably 5% by mass or more and 18% by mass or less and still more preferably 8% by mass or more and 16% by mass or less.

### "Disperser"

The disperser is not particularly limited as long as the disperser is capable of imparting dispersion energy to the liquid mixture to an extent that the surface treatment can be performed while loosening the agglomeration of the zinc oxide particles. The kind or description of the disperser described in the wet treatment of the first embodiment can also be preferably used in the present step of the present embodiment.

In the present step, the liquid mixture is dispersed using the disperser with a predetermined amount of energy or more to obtain surface-modified zinc oxide particles.

The energy that is imparted to the liquid mixture may be appropriately adjusted according to the size of the disperser. Therefore, the conditions of the dispersion may be selected as appropriate. For example, in a case where the dispersion treatment is performed with a mill, specifically, a bead mill using a container having a capacity of about 1 L, it is preferable to perform the dispersion treatment at a rotation speed of 500 rpm or faster for 1 hour or longer and 10 hours or shorter. Here, the conditions can be selected as necessary and are not limited only to the above conditions.

In addition, in a case where the dispersion treatment is performed with a mill, specifically, a bead mill using a container having a capacity of about 1 L, the dispersion energy that is imparted to the liquid mixture is, for example, preferably 100 W-h/kg or higher and 600 W-h/kg or lower. Here, the conditions can be selected as necessary and are not limited only to the above conditions.

In addition, since the cracking force of the bead mill depends on the centrifugal force of a disc or beads on the pin outer periphery in the mill, when the product (impulse) of the centrifugal force and the dispersion time is calculated in a case where the total weight of the beads in the mill is regarded as the bead weight, the dispersion treatment is preferably performed such that the product becomes 0.5 × 10⁶ N.s or higher and 100 × 10⁶ N.s or lower.

In addition, the dispersion treatment may be performed until b* of the surface-modified zinc oxide particles becomes 4.0 or higher and 18 or lower. In this case, the degree of progress of the dispersion treatment may be confirmed by extracting a small amount of the zinc oxide particles during the dispersion treatment and measuring b* of the zinc oxide particles using a spectral colorimeter.

The temperature in the step of dispersing the liquid mixture with the disperser is not particularly limited, but is, for example, preferably 20°C or higher and 45°C or lower.

### "Third step of preparing second mixture (second surface treatment step)"

The present step is a step of mixing the first mixture obtained in the second step and the hydrolysate again to produce a second mixture, thereby performing the surface treatment of the zinc oxide particles with the surface treatment agent one more time.

The surface treatment performed twice makes it easy for the surface treatment agent to be uniformly attached to the particle surfaces. The surface treatment step may be performed three times, four times, or five or more times. That is, the surface treatment step can be repeated once or more times until intended particles are obtained.

The mixing ratio between the hydrolysate and the zinc oxide particles is not particularly limited, but it is preferable to mix the hydrolysate and the zinc oxide particles so that the surface treatment agent becomes 1% by mass or more and 20% by mass or less in the total mass of the zinc oxide particles and the surface treatment agent after the second step. The mixing ratio is more preferably 5% by mass or more and 18% by mass or less and still more preferably 8% by mass or more and 16% by mass or less.

The mass ratio between the surface treatment agent mixed in the second step and the surface treatment agent mixed in the third step is not particularly limited, but is preferably 0.5:1 to 3:1. The mass ratio may be 0.6:1 to 2:1, 0.7:1 to 1.5:1, or the like as necessary.

Since the second and subsequent surface treatment steps are performed to suppress the remaining of hydrolyzable reactive groups, it is preferable to reduce the mixing ratio of the surface treatment agent and accelerate the surface treatment by heating or the like.

Since the present step is a step performed to suppress the remaining of hydrolyzable reactive groups, the first mixture and the hydrolysate are preferably mixed while being heated.

That is, in the case of performing the surface treatment in a wet manner, the surface treatment agent is uniformly attached to the surfaces of the zinc oxide particles in the second step, and the surface treatment is further accelerated in the third step.

The heating temperature is not particularly limited as long as the surface treatment is accelerated at the heating temperature and is, for example, preferably 40°C or higher and 150°C or lower. The heating temperature may be 40°C or higher and 80°C or lower, 60°C to 100°C, or the like as necessary.

### "Drying step"

The present step is performed to remove water and the solvent remaining in the second mixture. In the present step, it is preferable to dry the second mixture using a drying device. A treatment that shortens the drying time, such as solid-liquid separation, may be performed before drying. The drying device is not particularly limited, and, for example, the drying device described in the drying step of the wet treatment of the first embodiment can also be preferably used in the present step.

The drying temperature is not particularly limited as long as the solvent can be removed, but is preferably, for example, 50°C or higher and 200°C or lower. The drying temperature may be 60°C or higher and 150°C or lower, 70°C or higher and 120°C or lower, or the like.

### "Cracking step"

The dried surface-modified zinc oxide particles may be cracked using a cracking device. The cracking device can be arbitrarily selected, and examples thereof include an atomizer, a hammer mill, a jet mill, an impeller mill, a pin mill, and the like. The cracking step makes it possible to suppress the rough feel of the surface-modified zinc oxide particles when, for example, the surface-modified zinc oxide particles are blended with cosmetic preparations. That is, the cracking step makes it possible to improve usability when the surface-modified zinc oxide particles are used in cosmetic preparations.

The above-described steps make it possible to produce the surface-modified zinc oxide particles of the present embodiment.

### [Dispersion liquid]

A dispersion liquid of the present embodiment contains either or both of the surface-modified zinc oxide particles of the first and second embodiments and a dispersion medium. The dispersion liquid may be formed by preparing the surface-modified zinc oxide particles of the present embodiment and mixing the surface-modified zinc oxide particles with a dispersion medium.

The dispersion liquid of the present embodiment may also preferably include a paste-form dispersing element having a high viscosity.

The dispersion medium is not particularly limited as long as the surface-modified zinc oxide particles can be dispersed. In a case where the surface-modified zinc oxide particles are used for cosmetic preparations, the dispersion medium is not particularly limited as long as the dispersion medium can be prescribed for cosmetic preparations.

Examples of the dispersion medium include hydrophobic dispersion media such as chain-like polysiloxanes such as dimethyl polysiloxane, methylphenyl polysiloxane, and diphenylpolysiloxane; cyclic polysiloxanes such as octamethylcyclotetrasiloxane, decamethylcyclopentasiloxane, and dodecamethyl cyclohexasiloxane; modified polysiloxane such as amino-modified polysiloxane, polyether-modified polysiloxane, alkyl-modified polysiloxane, and fluorine-modified polysiloxane, hydrocarbon oils such as liquid paraffin, squalane, isoparaffin, branched chain-like light paraffin, vaseline, ceresin, dodecane, isododecane, tridecane, tetradecane, hexadecane, isohexadecane, and octadecane; ester oils such as isopropyl myristate, cetyl isooctanoate, glyceryl trioctanoate, tri(caprylic acid/capric acid) glyceryl, and alkyl benzoate (C12-15); higher fatty acids such as lauric acid, myristic acid, palmitic acid, and stearic acid; higher alcohols such as lauryl alcohol, cetyl alcohol, stearyl alcohol, octyldodecanol, and isostearyl alcohol; and the like. In addition, examples thereof include alcohols such as methanol, ethanol, n-propanol, isopropanol, n-butanol, 2-butanol, octanol, and glycerin; esters such as ethyl acetate, butyl acetate, ethyl lactate, propylene glycol monomethyl ether acetate, propylene glycol monoethyl ether acetate, and γ-butyrolactone; ethers such as diethyl ether, ethylene glycol monomethyl ether (methyl cellosolve), ethylene glycol monoethyl ether (ethyl cellosolve), ethylene glycol monobutyl ether (butyl cellosolve), diethylene glycol monomethyl ether, and diethylene glycol monoethyl ether; aromatic hydrocarbons such as benzene, toluene, ethylbenzene, 1-phenylpropane, isopropylbenzene, n-butylbenzene, tert-butylbenzene, sec-butylbenzene, o-, m-, or p-xylene, and 2-, 3- or 4-ethyltoluene; ketones such as acetone, methyl ethyl ketone, methyl isobutyl ketone, and cyclohexanone; amides such as dimethylformamide, N,N-dimethylacetoacetamide, and N-methylpyrrolidone; nitriles such as acetonitrile; natural oils such as oleic acid, jojoba oil, olive oil, coconut oil, grapeseed oil, castor oil, rice bran oil, horse oil, and mink oil; and the like.

These dispersion media may be used singly or two or more dispersion media may be used in a mixture form.

In a case where the surface-modified zinc oxide particles are used for cosmetic preparations, as the dispersion medium, the above chain-like polysiloxane, the above cyclic polysiloxane, the above modified polysiloxane, the above hydrocarbon oil, the above ester oil, the above higher fatty acid, the above higher alcohol, the above natural oil, ethanol, glycerin, and the like are preferably used.

These dispersion media may be used singly or two or more dispersion media may be used in a mixture form.

The dispersion liquid of the present embodiment may include a commonly-used additive as long as the characteristic thereof is not impaired.

Examples of the additive include a preservative, a dispersant, a dispersion aid, a stabilizer, a water-soluble binder, a viscosity improver, an oil-soluble chemical, oil-soluble pigments, oil-soluble proteins, an UV absorber, and the like.

The particle diameter (d50) when the cumulative volume percentage of the particle size distribution in the dispersion liquid of the present embodiment is 50% is arbitrarily selected, but is preferably 300 nm or less (0.3 um or less), more preferably 250 nm or less, and still more preferably 200 nm or less. The upper limit value of d50 may be 150 nm or less or 100 nm or less.

The lower limit value of d50 is not particularly limited and may be, for example, 20 nm or more, may be 40 nm or more, or may be 60 nm or more.

The upper limit value and lower limit value of d50 can be arbitrarily combined together.

In addition, the particle diameter (d90) when the cumulative volume percentage of the particle size distribution in the dispersion liquid of the present embodiment is 90% is preferably 350 nm or less, more preferably 300 nm or less, and still more preferably 250 nm or less.

The lower limit value of d90 is not particularly limited and may be, for example, 60 nm or more, may be 80 nm or more, or may be 100 nm or more.

The upper limit value and lower limit value of d90 can be arbitrarily combined together.

In a case where d50 of the dispersion liquid is 300 nm or less, when a cosmetic preparation produced using this dispersion liquid has been applied to the skin, it is easy to uniformly distribute the surface-modified zinc oxide particles and an ultraviolet shielding effect improves, which is preferable. In addition, in a case where d90 of the dispersion liquid is 350 nm or less, the transparency of the dispersion liquid is high, and the transparency of cosmetic preparations produced using this dispersion liquid also becomes high, which is preferable.

That is, when d50 and d90 in the dispersion liquid of the present embodiment are within the above-described ranges, it is possible to obtain a dispersion liquid having excellent transparency and excellent ultraviolet shielding properties. In addition, cosmetic preparations produced using this dispersion liquid are also excellent in terms of transparency and ultraviolet shielding properties.

Examples of a method for measuring the cumulative volume percentage of the particle size distribution in the dispersion liquid of the present embodiment include a method in which a dynamic light scattering particle size distribution analyzer (manufactured by MicrotracBEL Corp., model number: NANOTRAC WAVE) is used.

The content of the surface-modified zinc oxide particles in the dispersion liquid of the present embodiment is appropriately adjusted depending on the intended characteristics of the dispersion liquid.

In the case of using the dispersion liquid of the present embodiment in a cosmetic preparation, the content of the surface-modified zinc oxide particles in the dispersion liquid is preferably 30% by mass or more, more preferably 40% by mass or more, and still more preferably 50% by mass or more. In addition, the content of the surface-modified zinc oxide particles in the dispersion liquid is preferably 90% by mass or less, more preferably 85% by mass or less, and still more preferably 80% by mass or less.

The upper limit value and lower limit value of the content of the surface-modified zinc oxide particles in the dispersion liquid can be arbitrarily combined together.

When the content of the surface-modified zinc oxide particles in the dispersion liquid is within the above range, the surface-modified zinc oxide particles are contained at a high concentration in the dispersion liquid. Therefore, it is possible to improve the degree of freedom in formulation of cosmetic preparations that are produced using the dispersion liquid and to adjust the viscosity of the dispersion liquid to a range where handling is easy.

A method for producing the dispersion liquid of the present embodiment is not particularly limited. Examples thereof include a method in which the surface-modified zinc oxide particles of the present embodiment and a dispersion medium are mechanically dispersed with a well-known dispersion device.

The dispersion device can be selected as necessary. Examples of the dispersion device include a stirrer, a planetary mixer, a homogenizer, an ultrasonic homogenizer, a sand mill, a ball mill, a roll mill, and the like.

The dispersion liquid of the present embodiment can be used for, in addition to cosmetic preparations, paints and the like having an ultraviolet shielding function, a gas transmission-suppressing function, or the like.

The dispersion liquid of the present embodiment contains the surface-modified zinc oxide particles of the present embodiment and thus has excellent ultraviolet shielding properties.

### [Composition]

A composition of the present embodiment contains either or both of the surface-modified zinc oxide particles of the first and second embodiments, a resin, and a dispersion medium. The composition may be prepared by preparing the surface-modified zinc oxide particles of the present embodiment and mixing the surface-modified zinc oxide particles with a resin and a dispersion medium.

The content of the surface-modified zinc oxide particles in the composition of the present embodiment is appropriately adjusted depending on the intended characteristics of the composition. The content of the surface-modified zinc oxide particles in the composition of the present embodiment is, for example, preferably 10% by mass or more and 40% by mass or less and more preferably 20% by mass or more and 30% by mass or less.

When the content of the surface-modified zinc oxide particles in the composition is within the above range, the surface-modified zinc oxide particles are contained at a high concentration in the composition. Therefore, the characteristics of the surface-modified zinc oxide particles can be sufficiently obtained, and a composition in which the surface-modified zinc oxide particles are uniformly dispersed can be obtained.

The dispersion medium is not particularly limited as long as the dispersion medium is commonly used for industrial applications. Examples of the dispersion medium include alcohols such as methanol, ethanol, and propanol, methyl acetate, ethyl acetate, toluene, methyl ethyl ketone, methyl isobutyl ketone, and the like.

The content of the dispersion medium in the composition of the present embodiment is not particularly limited and is appropriately adjusted depending on the intended characteristics of the composition.

The resin is not particularly limited as long as the resin is commonly used for industrial applications. Examples of the resin include an acrylic resin, an epoxy resin, a urethane resin, a polyester resin, a silicone resin, and the like.

The content of the resin in the composition of the present embodiment is not particularly limited and is appropriately adjusted depending on the intended characteristics of the composition.

The composition of the present embodiment may contain a commonly-used additive to an extent that the characteristics thereof are not impaired.

Examples of the additives include a polymerization initiator, a dispersant, a preservative, and the like.

A method for producing the composition of the present embodiment is not particularly limited. Examples thereof include a method in which the surface-modified zinc oxide particles of the present embodiment, a resin, and a dispersion medium are mechanically mixed with a well-known mixing device.

In addition, examples thereof include a method in which the above-described dispersion liquid and the resin are mechanically mixed together using a well-known mixing device.

Examples of the mixing device include a stirrer, a planetary mixer, a homogenizer, an ultrasonic homogenizer, and the like.

A coated film can be formed by applying the composition of the present embodiment to a plastic base material such as a polyester film using a normal application method such as a roll coating method, a flow coating method, a spray coating method, a screen printing method, a brush coating method, or an immersion method. The coated film can be used as an ultraviolet shielding film or a gas barrier film.

The composition of the present embodiment contains the surface-modified zinc oxide particles of the present embodiment and thus has excellent ultraviolet shielding properties.

### [Cosmetic preparation]

A cosmetic preparation of the present embodiment contains at least one of the surface-modified zinc oxide particles of the present embodiment and the dispersion liquid of the present embodiment and a cosmetic base raw material.

Here, the cosmetic base raw material refers to various raw materials that form the main body of the cosmetic product, and examples thereof include an oily raw material, an aqueous raw material, a surfactant, a powder raw material, and the like.

Examples of the oily raw material include fats and oils, higher fatty acids, higher alcohols, ester oils, and the like.

Examples of the aqueous raw material include purified water, alcohol, a viscosity improver, and the like.

Examples of the powder raw material include a colored pigment, a white pigment, a pearl agent, an extender pigment, and the like.

In the present embodiment, as the cosmetic base raw material, an oily raw material, a powder raw material, or an oily raw material and a powder raw material can be preferably used, and an oily raw material can be more preferably used.

The cosmetic preparation of the present embodiment may mean a cosmetic preparation where surface-modified zinc oxide particles are contained in an oil component (oil phase) in the production process or in the final form such as an oily cosmetic preparation, an emulsion type cosmetic preparation containing surface-modified zinc oxide particles in an oil phase, or a powdery solid cosmetic preparation produced by mixing surface-modified zinc oxide particles and an oil, then, removing the oil and forming the surface-modified zinc oxide particles.

The emulsion type cosmetic preparation may be an O/W type emulsion or may be a W/O type emulsion.

In other words, the cosmetic preparation of the present embodiment preferably contains at least one of the surface-modified zinc oxide particles of the present embodiment and the dispersion liquid of the present embodiment in an oil component or an oil phase.

The oil component that is used in the oily cosmetic preparation or the oil phase of emulsion is not particularly limited as long as the oil component is commonly used in cosmetic preparations. Examples thereof include silicone oil, oil and fat, higher fatty acid, higher alcohol, ester oil, natural oil, and the like.

In addition, the cosmetic preparation of the present embodiment may contain the above-described aqueous raw material, surfactant, powder raw material, and the like to an extent that the characteristics of the cosmetic preparation are not impaired.

The cosmetic preparation of the present embodiment can be obtained by, for example, blending the surface-modified zinc oxide particles of the present embodiment or the dispersion liquid of the present embodiment with cosmetic base raw materials such as an emulsion, a cream, a sunscreen, a foundation, a lip stick, a blush, or an eye shadow as in the related art.

In addition, the cosmetic preparation of the present embodiment can be obtained by blending the surface-modified zinc oxide particles of the present embodiment with an oil phase to produce an O/W type or W/O type emulsion and blending the emulsion with the cosmetic base raw materials.

The content of the surface-modified zinc oxide particles in the cosmetic preparation of the present embodiment is appropriately adjusted depending on the intended characteristics of the cosmetic preparation. For example, the lower limit of the content of the surface-modified zinc oxide particles may be 0.01% by mass or more, may be 0.1% by mass or more, or may be 1% by mass or more. In addition, the upper limit of the content of the surface-modified zinc oxide particles may be 50% by mass or less, may be 40% by mass or less, or may be 30% by mass or less.

The upper limit value and lower limit value of the content of the surface-modified zinc oxide particles in the cosmetic preparation can be arbitrarily combined together.

Hereinafter, as an example of the cosmetic preparation, a sunscreen cosmetic preparation will be specifically described.

In order to effectively shield ultraviolet rays, particularly, long wavelength ultraviolet rays (UVA) and to obtain a favorable usability with small powdery squeak, the lower limit of the content of the surface-modified zinc oxide particles in the sunscreen cosmetic preparation is preferably 0.01% by mass or more, more preferably 0.1% by mass or more, and still more preferably 1% by mass or more. In addition, the upper limit of the content of the surface-modified zinc oxide particles in the sunscreen cosmetic preparation may be 50% by mass or less, may be 40% by mass or less, or may be 30% by mass or less. The upper limit value and lower limit value of the content of the surface-modified zinc oxide particles in the sunscreen cosmetic preparation can be arbitrarily combined together.

The sunscreen cosmetic preparation may contain a hydrophobic dispersion medium, inorganic fine particles or inorganic pigment other than surface-modified zinc oxide particles, a hydrophilic dispersion medium, fats and oils, a surfactant, a moisturizer, a viscosity improver, a pH adjuster, a nutrient, an antioxidant, a fragrance, a preservative, a dispersant, an antifoaming agent, a coloring agent, a cosmetic ingredient, a polymeric substance, a bio-derived ingredient, a plant-derived ingredient, an antibacterial agent, a bactericide, a fungicide, an aqueous Ingredient, an oily ingredient, a vitamin supplement, an emulsifier, a stabilizer, a solubilizer, a pearlescent agent, a refatting substance, and the like as necessary.

Examples of the hydrophobic dispersion medium include a hydrocarbon oil such as liquid paraffin, squalane, isoparaffin, branched chain-like light paraffin, vaseline, or ceresin, an ester oil such as isopropyl myristate, cetyl isooctanoate, or glyceryl trioctanoate, a silicone oil such as decamethyl cyclopentasiloxane, dimethyl polysiloxane, or methylphenyl polysiloxane, a higher fatty acid such as lauric acid, myristic acid, palmitic acid, or stearic acid, and a higher alcohol such as lauryl alcohol, cetyl alcohol, stearyl alcohol, hexyl dodecanol, or isostearyl alcohol, and the like.

Examples of the inorganic fine particles or inorganic pigment other than the surface-treated particles that are contained in the cosmetic preparation include calcium carbonate, calcium phosphate (apatite), magnesium carbonate, calcium silicate, magnesium silicate, aluminum silicate, kaolin, talc, titanium oxide, aluminum oxide, yellow oxide of iron, γ-iron oxide, cobalt titanate, cobalt violet, silicon oxide, and the like.

The sunscreen cosmetic preparation may further contain at least one organic ultraviolet absorber. The cosmetic preparation containing both the surface-modified zinc oxide particles and the organic ultraviolet absorber is preferable since the cosmetic preparation broadens the ultraviolet shielding region through a booster effect and also enhances ultraviolet shielding properties.

Examples of the organic ultraviolet absorber include a benzotriazole-based ultraviolet absorber, a benzoyl methane-based ultraviolet absorber, a benzoic acid-based ultraviolet absorber, an anthranilic acid-based ultraviolet absorber, a salicylic acid-based ultraviolet absorber, a cinnamic acid-based ultraviolet absorber, a silicone-based cinnamic acid ultraviolet absorber, a triazine-based ultraviolet absorber, and the like.

Examples of the benzotriazole-based ultraviolet absorber include 2,2'-hydroxy-5-methylphenylbenzotriazole, 2-(2'-hydroxy-5'-t-octylphenyl)benzotriazole, 2-(2'-hydroxy-5'-methylphenylbenzotriazole, and the like.

Examples of the benzoyl methane-based ultraviolet absorber include dibenzalazine, dianisoylmethane, 4-tert-butyl-4'-methoxydibenzoylmethane, 1-(4'-isopropylphenyl)-3-phenyl propane-1,3-dione, 5-(3,3'-dimethyl-2-norbornylidene)-3-pentane-2-one, and the like.

Examples of the benzoic acid-based ultraviolet absorber include para-aminobenzoic acid (PABA), PABA monoglycerin ester, N,N-dipropoxy PABA ethyl ester, N,N-diethoxy PABA ethyl ester, N,N-dimethyl PABA ethyl ester, N,N-dimethyl PABA butyl ester, N,N-dimethyl PABA methyl ester, and the like.

Examples of the anthranilic acid-based ultraviolet absorber include homo menthyl-N-acetyl anthranilate and the like.

Examples of the salicylic acid-based ultraviolet absorber include amyl salicylate, menthyl salicylate, homo menthyl salicylate, octyl salicylate, phenyl salicylate, benzyl salicylate, p-2-propanol phenyl salicylate, and the like.

Examples of the cinnamic acid-based ultraviolet absorber include octyl methoxycinnamate(ethylhexyl methoxycinnamate), di-para methoxy cinnamate-mono-2-glyceryl ethylhexanoate, octyl cinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxy cinnamate, isopropyl-p-methoxy cinnamate, isoamyl-p-methoxy cinnamate, octyl-p-methoxy cinnamate(2-ethylhexyl-p-methoxy cinnamate), 2-ethoxyethyl-p-methoxy cinnamate, cyclohexyl-p-methoxy cinnamate, ethyl-α-cyano-β-phenyl cinnamate, 2-ethylhexyl-α-cyano-β-phenyl cinnamate, glyceryl mono-2-ethylhexanoyl-diparamethoxy cinnamate, and the like.

Examples of the silicone-based cinnamic acid ultraviolet absorber include [3-bis(trimethylsiloxy)methylsilyl-1-methylpropyl]-3,4,5-trimethoxy cinnamate, [3-bis(trimethylsiloxy)methylsilyl-3-methylpropyl]-3,4,5-trimethoxy cinnamate, [3-bis(trimethylsiloxy)methylsilylpropyl]-3,4,5-trimethoxy cinnamate, [3-bis(trimethylsiloxy)methylsilylbutyl]-3,4,5-trimethoxy cinnamate, [3-tris(trimethylsiloxy)silylbutyl]-3,4,5-trimethoxy cinnamate, [3-tris(trimethylsiloxy)silyl-1-methylpropyl]-3,4-dimethoxy cinnamate, and the like.

Examples of an example of the triazine-based ultraviolet absorber include bis-ethylhexyloxyphenol methoxyphenyl triazine, ethylhexyl triazone, methylene bis-benzotriazolyl tetramethylbutylphenol, tris-biphenyl triazine, diethylhexyl butamido triazone, and the like.

Examples of the organic ultraviolet absorbers other than the above-described ultraviolet absorbers include 3-(4'-methylbenzylidene)-d,l-camphor, 3-benzylidene-d,l-camphor, urocanic acid, ethyl urocanate esters, 2-phenyl-5-methylbenzoxane, 5-(3,3'-dimethyl-2-norbornylidene)-3-pentane-2-one, silicone-denatured ultraviolet absorbers, fluorine-denatured ultraviolet absorbers, and the like.

The cosmetic preparation of the present embodiment contains the surface-modified zinc oxide particles of the present embodiment and thus has excellent ultraviolet shielding properties.

### Examples

Hereinafter, the present invention will be more specifically described with examples and comparative examples, but the present invention is not limited to the following examples.

### [Example 1]

### "Production of surface-modified zinc oxide particles"

### (First step of preparing hydrolysate)

40.0 g of octyltriethoxysilane (manufactured by Shin-Etsu Chemical Co., Ltd., product name: KBE-3083), 8.0 g of pure water, 0.8 g of 0.1 mol/L hydrochloric acid, and 51.2 g of ethanol were mixed at 60°C for 90 minutes to obtain a hydrolysate.

### (Second step of preparing first mixture)

4 g of the obtained hydrolysate, 25 g of zinc oxide particles (manufactured by Sumitomo Osaka Cement Co., Ltd.) having a specific surface area of 36.4 m²/g, and 71 g of ethanol were mixed together.

Next, this liquid mixture was dispersed using a bead mill. As dispersion conditions, the rotation speed was set to 1300 rpm, the temperature was set to 20°C, and the dispersion time was set to 40 minutes. After a dispersion treatment, beads were removed, and a first mixture was obtained.

### (Third step of preparing second mixture)

100 g of the obtained first mixture and 3.0 g of the hydrolysate were mixed at 50°C for 30 minutes to obtain a second mixture.

### (Drying step)

The obtained second mixture was solid-liquid separated, dried at 100°C for 3 hours, and pulverized with a jet mill, thereby obtaining surface-modified zinc oxide particles of Example 1.

### "Measurement of specific surface area of surface-modified zinc oxide particles"

The specific surface area of the surface-modified zinc oxide particles of Example 1 was measured using a full automatic specific surface area-measuring instrument (trade name: Macsorb HM Model-1201, manufactured by Mountech Co., Ltd.). The result is shown in Table 1.

### "Measurement of color difference ΔE before and after irradiation with simulated sunlight of surface-modified zinc oxide particles"

3 g of t-butyl methoxydibenzoylmethane, 3 g of polyhydroxystearic acid, and 94 g of tri(caprylic acid/capric acid) glyceryl were mixed, and a solution in which t-butyl methoxydibenzoylmethane was completely dissolved was produced.

Next, 2.0 g of the obtained solution and 3.0 g of the surface-modified zinc oxide particles of Example 1 were mixed with a kneader (manufactured by THINKY Corporation, model number: ARE-310) to become uniform, thereby obtaining a liquid mixture.

Next, the obtained liquid mixture was sandwiched between assembled quartz cells for a spectrophotometer (manufactured by GL Sciences Inc., model number: AB20-UV-05), so that the optical path length became 0.5 mm, thereby producing an assembled cell.

Next, the obtained assembled cell was set in an ultraviolet-visible-near-infrared spectrophotometer (manufactured by JASCO Corporation, model number: V-770), the diffuse reflectance spectrum of the liquid mixture was measured using an integrating sphere, and the L₁*, a₁*, b₁* of the liquid mixture was calculated from the measurement results.

Next, the assembled cell containing the liquid mixture was irradiated with simulated sunlight having an accumulated light intensity of 300 kJ/m² using a xenon lamp of a small light irradiation test device (manufactured by Iwasaki Electric Co., Ltd., model number: EYE SUN-CUBE Xenon).

The irradiated assembled cell was set again in the ultraviolet-visible-near-infrared spectrophotometer, the diffuse reflectance spectrum was measured in the same manner as described above, and the L₂*, a₂*, b₂* of the liquid mixture after irradiation with simulated sunlight was calculated from the measurement results.

Next, the color difference ΔE = ((L₂* - L₁*)² + (a₂* - a₁*)² + (b₂* - b₁*)²)^{1/2} was calculated. The result is shown in Table 1.

### "Production of dispersion liquid"

10 g of the surface-modified zinc oxide particles of Example 1, 88 g of cyclopentasiloxane (manufactured by Dow Toray Co., Ltd., model number: DOWSIL SH 245 Fluid), and 2 g of polyglyceryl-3 polydimethylsiloxyethyl dimethicone (manufactured by Shin-Etsu Chemical Co., Ltd., model number: KF-6106) were mixed to obtain a liquid mixture.

Next, a dispersion treatment was performed on this liquid mixture at 9500 rpm for 5 minutes using a homogenizer (manufactured by IKA, ULTRA-TURRAX (registered trademark) series: T25 basic) to obtain a dispersion liquid of Example 1.

### "Evaluation of dispersibility by particle size distribution"

The dispersion liquid of Example 1 was diluted with cyclopentasiloxane such that the content of the surface-modified zinc oxide particles became 0.1% by mass to produce a measurement solution.

This measurement solution was used to measure the particle diameter (d10) when the cumulative volume percentage of the particle size distribution was 10%, the particle diameter (d50) when the cumulative volume percentage of the particle size distribution was 50%, and the particle diameter (d90) when the cumulative volume percentage of the particle size distribution was 90% using a dynamic light scattering particle size distribution analyzer (manufactured by MicrotracBEL Corp., model number: NANOTRAC WAVE). The results are shown in Table 2.

### "Evaluation of transparency and ultraviolet shielding properties of dispersion liquid"

The dispersion liquid of Example 1 was diluted with cyclopentasiloxane such that the concentration of the surface-modified zinc oxide particles became 0.005% by mass.

This diluted liquid was put into a quartz cell having an optical path length of 10 mm, and the total light transmittance (linear transmittance + diffuse transmittance) (%) at 360 nm and the linear transmittance (%) at 550 nm were measured using an ultraviolet-visible-near-infrared spectrophotometer (manufactured by JASCO Corporation, model number: V-770). The results are shown in Table 2.

A low transmittance at 360 nm indicates that the ultraviolet shielding properties are high. Therefore, the transmittance at 360 nm is preferably low.

A high transmittance at 550 nm indicates that the transparency is high. Therefore, the transmittance at 550 nm is preferably high.

### "Evaluation of temporal stability of surface-modified zinc oxide particles"

The surface-modified zinc oxide particles of Example 1 were put into a porcelain evaporating dish, covered with a polyimide film, and exposed to an environment of 85°C and 95 %RH for 48 hours. A dispersion liquid was produced using the exposed surface-modified zinc oxide particles of Example 1 in the same manner as described above. The particle size distribution, the transparency, and the ultraviolet shielding properties were measured in the same manner as described above using the obtained dispersion liquid. The results are shown in Table 2.

### [Example 2]

Surface-modified zinc oxide particles of Example 2 were obtained in the same manner as in Example 1 except that, in the second step of preparing the mixture of Example 1, zinc oxide particles having a specific surface area of 19.2 m²/g (manufactured by Sumitomo Osaka Cement Co., Ltd.) were used instead of the use of the zinc oxide particles having a specific surface area of 36.4 m²/g, 2.4 g of the hydrolysate and 72.6 g of ethanol were used instead of using 4 g of the hydrolysate and 71 g of ethanol, and, in the third step of preparing the second mixture of Example 1, 1.6 g of the hydrolysate was used instead of 3 g of the hydrolysate.

The specific surface area and color difference ΔE of the surface-modified zinc oxide particles of Example 2 were measured in the same manner as in Example 1. The result is shown in Table 1.

A dispersion liquid of Example 2 was obtained in the same manner as in Example 1 except that the surface-modified zinc oxide particles of Example 2 were used instead of the use of the surface-modified zinc oxide particles of Example 1.

The particle size distribution, transparency, and ultraviolet shielding properties of the dispersion liquid of Example 2 were evaluated in the same manner as in Example 1. The results are shown in Table 2.

The surface-modified zinc oxide particles of Example 2 were exposed to an environment of 85°C and 95 %RH for 48 hours in the same manner as in Example 1. The exposed surface-modified zinc oxide particles of Example 2 were evaluated in the same manner as in Example 1. The results are shown in Table 2.

### [Example 3]

Surface-modified zinc oxide particles of Example 3 were obtained in the same manner as in Example 1 except that, in the second step of preparing the mixture of Example 1, zinc oxide particles having a specific surface area of 4.7 m²/g (manufactured by Sumitomo Osaka Cement Co., Ltd.) were used instead of the use of the zinc oxide particles having a specific surface area of 36.4 m²/g, 1.2 g of the hydrolysate and 73.8 g of ethanol were used instead of using 4 g of the hydrolysate and 71 g of ethanol, and, in the third step of preparing the second mixture of Example 1, 0.8 g of the hydrolysate was used instead of 3 g of the hydrolysate.

The specific surface area and color difference ΔE of the surface-modified zinc oxide particles of Example 3 were measured in the same manner as in Example 1. The result is shown in Table 1.

A dispersion liquid of Example 3 was obtained in the same manner as in Example 1 except that the surface-modified zinc oxide particles of Example 3 were used instead of the use of the surface-modified zinc oxide particles of Example 1.

The particle size distribution, transparency, and ultraviolet shielding properties of the dispersion liquid of Example 3 were evaluated in the same manner as in Example 1. The results are shown in Table 2.

The surface-modified zinc oxide particles of Example 3 were exposed to an environment of 85°C and 95 %RH for 48 hours in the same manner as in Example 1. The exposed surface-modified zinc oxide particles of Example 3 were evaluated in the same manner as in Example 1. The results are shown in Table 2.

### [Example 4]

### (Second step of preparing first mixture)

7 g of the hydrolysate obtained in the first step of Example 1, 25 g of zinc oxide particles (manufactured by Sumitomo Osaka Cement Co., Ltd.) having a specific surface area of 38.7 m²/g, and 68 g of ethanol were mixed together.

Next, this liquid mixture was dispersed using a bead mill. As dispersion conditions, the rotation speed was set to 1300 rpm, the temperature was set to 20°C, and the dispersion time was set to 40 minutes. After a dispersion treatment, beads were removed, and a first mixture was obtained.

### (Drying step)

The obtained first mixture was solid-liquid separated and dried at 100°C for 3 hours, thereby obtaining surface-modified zinc oxide particles of Example 4.

The specific surface area and color difference ΔE of the surface-modified zinc oxide particles of Example 4 were measured in the same manner as in Example 1. The result is shown in Table 1.

A dispersion liquid of Example 4 was obtained in the same manner as in Example 1 except that the surface-modified zinc oxide particles of Example 4 were used instead of the use of the surface-modified zinc oxide particles of Example 1.

The particle size distribution, transparency, and ultraviolet shielding properties of the dispersion liquid of Example 4 were evaluated in the same manner as in Example 1. The results are shown in Table 2.

The surface-modified zinc oxide particles of Example 4 were exposed to an environment of 85°C and 95 %RH for 48 hours in the same manner as in Example 1. The exposed surface-modified zinc oxide particles of Example 4 were evaluated in the same manner as in Example 1. The results are shown in Table 2.

### [Example 5]

42 g of the hydrolysate obtained in the first step of Example 1 and 250 g of zinc oxide particles (manufactured by Sumitomo Osaka Cement Co., Ltd.) having a specific surface area of 39.4 m²/g were injected into a Henschel mixer and stirred and mixed for 1 hour.

Next, this mixture was dried at 80°C for 1 hour.

250 g of the dried surface-modified zinc oxide particles and 28 g of the hydrolysate obtained in the first step of Example 1 were injected into a Henschel mixer and stirred and mixed for 1 hour.

The obtained mixture was pulverized with a jet mill, and this pulverized powder was dried at 100°C for 3 hours, thereby obtaining surface-modified zinc oxide particles of Example 5.

The specific surface area and color difference ΔE of the surface-modified zinc oxide particles of Example 5 were measured in the same manner as in Example 1. The result is shown in Table 1.

A dispersion liquid of Example 5 was obtained in the same manner as in Example 1 except that the surface-modified zinc oxide particles of Example 5 were used instead of the use of the surface-modified zinc oxide particles of Example 1.

The particle size distribution, transparency, and ultraviolet shielding properties of the dispersion liquid of Example 5 were evaluated in the same manner as in Example 1. The results are shown in Table 2.

The surface-modified zinc oxide particles of Example 5 were exposed to an environment of 85°C and 95 %RH for 48 hours in the same manner as in Example 1. The exposed surface-modified zinc oxide particles of Example 5 were evaluated in the same manner as in Example 1. The results are shown in Table 2.

### [Example 6]

69 g of the hydrolysate obtained in the first step of Example 1 and 250 g of zinc oxide particles (manufactured by Sumitomo Osaka Cement Co., Ltd.) having a specific surface area of 39.4 m²/g were injected into a Henschel mixer and stirred and mixed for 1 hour.

The obtained mixture was pulverized with a jet mill, and this pulverized powder was dried at 100°C for 3 hours, thereby obtaining surface-modified zinc oxide particles of Example 6.

The specific surface area and color difference ΔE of the surface-modified zinc oxide particles of Example 6 were measured in the same manner as in Example 1. The result is shown in Table 1.

A dispersion liquid of Example 6 was obtained in the same manner as in Example 1 except that the surface-modified zinc oxide particles of Example 6 were used instead of the use of the surface-modified zinc oxide particles of Example 1.

The particle size distribution, transparency, and ultraviolet shielding properties of the dispersion liquid of Example 6 were evaluated in the same manner as in Example 1. The results are shown in Table 2.

The surface-modified zinc oxide particles of Example 6 were exposed to an environment of 85°C and 95 %RH for 48 hours in the same manner as in Example 1. The exposed surface-modified zinc oxide particles of Example 6 were evaluated in the same manner as in Example 1. The results are shown in Table 2.

### [Comparative Example 1]

100 g of zinc oxide particles (manufactured by Sumitomo Osaka Cement Co., Ltd.) having a specific surface area of 39.4 m²/g were put into a Henschel mixer. While the zinc oxide particles were stirred with the Henschel mixer, a liquid mixture of 6 g of octyltriethoxysilane (trade name: KBE-3083, manufactured by Shin-Etsu Chemical Co., Ltd.), 0.45 g of pure water, and 8.55 g of isopropyl alcohol was added thereto. The mixture was mixed in the Henschel mixer and stirred and mixed for 1 hour.

Next, the obtained mixture was pulverized with a jet mill, and the pulverized powder was dried at 100°C for 3 hours to obtain surface-modified zinc oxide particles of Comparative Example 1.

The specific surface area and color difference ΔE of the surface-modified zinc oxide particles of Comparative Example 1 were measured in the same manner as in Example 1. The result is shown in Table 1.

A dispersion liquid of Comparative Example 1 was obtained in the same manner as in Example 1 except that the surface-modified zinc oxide particles of Comparative Example 1 were used instead of the use of the surface-modified zinc oxide particles of Example 1.

The particle size distribution, transparency, and ultraviolet shielding properties of the dispersion liquid of Comparative Example 1 were evaluated in the same manner as in Example 1. The results are shown in Table 2.

The surface-modified zinc oxide particles of Comparative Example 1 were exposed to an environment of 85°C and 95 %RH for 48 hours in the same manner as in Example 1. The exposed surface-modified zinc oxide particles of Comparative Example 1 were evaluated in the same manner as in Example 1. The results are shown in Table 2.

### [Comparative Example 2]

The color difference ΔE was evaluated in the same manner as in Example 1 using the zinc oxide particles (manufactured by Sumitomo Osaka Cement Co., Ltd.) having a specific surface area of 39.4 m²/g used in Example 5, that is, zinc oxide particles that were not surface-treated as Comparative Example 2. The result is shown in Table 1.

**[Table 1]**

| | Example 1 | Example 2 | Example 3 | Example 4 | Example 5 | Example 6 | Comparative Example 1 | Comparative Example 2 |
|---|---|---|---|---|---|---|---|---|
| Specific surface area of zinc oxide particles [m²/g] | 36.4 | 19.2 | 4.7 | 38.7 | 39.4 | 39.4 | 39.4 | 39.4 |
| Zinc oxide: OTS (mass ratio) | 90:10 | 94:6 | 97:3 | 90:10 | 90 : 10 | 90: 10 | 94.3:5.7 | 100:0 |
| Surface treatment method | Wet manner | Wet manner | Wet manner | Wet manner | Dry manner | Dry manner | Dry manner | - |
| Number of times of mixing with hydrolysate | 2 | 2 | 2 | 1 | 2 | 1 | 0 | - |
| Specific surface area of surface-modified zinc oxide particles [m²/g] | 30.7 | 17.7 | 19.7 | 34.9 | 30.2 | 31.5 | 32.3 | - |
| ΔE | 1.5 | 1.6 | 1.8 | 1.8 | 0.2 | 0.2 | 9.7 | 16.3 |

**[Table 2]**

| | | Example 1 | Example 2 | Example 3 | Example 4 | Example 5 | Example 6 | Comparative Example 1 | Comparative Example 2 |
|---|---|---|---|---|---|---|---|---|---|
| Before exposure | D10 [µm] | 0.06 | 0.05 | 0.13 | 0.12 | 0.10 | 0.08 | 0.07 | - |
| | D50 [µm] | 0.12 | 0.09 | 0.18 | 0.18 | 0.22 | 0.19 | 0.14 | - |
| | D90 [µm] | 0.21 | 0.15 | 0.26 | 0.37 | 0.44 | 0.80 | 1.40 | - |
| | Transmittance at 360 nm [4] | 33.9 | 28.2 | 29.3 | 37.4 | 44.3 | 47.6 | 49.4 | - |
| | Transmittance at 550 nm [4] | 96.9 | 97.0 | 87.6 | 91.4 | 84.8 | 83.8 | 86.3 | - |
| After exposure | D10 [pm] | 0.09 | 0.05 | 0.17 | 0.09 | 0.08 | 0.10 | 0.17 | - |
| | D50 [pm] | 0.15 | 0.10 | 0.27 | 0.15 | 0.16 | 0.17 | 0.89 | - |
| | D90 [pm] | 0.26 | 0.17 | 0.39 | 0.38 | 0.38 | 0.70 | 1.65 | - |
| | Transmittance at 360 nm [&] | 35.6 | 28.7 | 47.5 | 48.8 | 42.5 | 48.2 | 69.3 | - |
| | Transmittance at 550 nm [4] | 95.8 | 96.3 | 76.3 | 89.1 | 86.4 | 83.9 | 75.0 | - |

From the results in Tables 1 and 2, it was confirmed that the surface-modified zinc oxide particles of Examples 1 to 6 have a small color difference ΔE and excellent ultraviolet shielding properties compared to those of the surface-modified zinc oxide particles of Comparative Example 1. In addition, it was also confirmed that the surface-modified zinc oxide particles surface-treated by the wet method have small d90, excellent transparency, and excellent ultraviolet shielding properties. In addition, it was confirmed from the comparison between Examples 1 to 3 and Examples 4 and 5 that the surface-modified zinc oxide particles having superior transparency and ultraviolet shielding properties were obtained by performing the mixing step with the hydrolysate by the wet method twice. In addition, it was confirmed from the comparison between Examples 1 and 2 and Example 3 that the deterioration of the surface-modified zinc oxide particles was suppressed even after the accelerated test by increasing the amount of the surface treatment agent.

### Industrial Applicability

The present invention is capable of providing surface-modified zinc oxide particles having excellent ultraviolet shielding properties. The surface-modified zinc oxide particles of the present invention have excellent ultraviolet shielding properties, also have transparency equal to or higher than that of conventional surface-modified zinc oxide particles, and are thus highly valuable industrially in the case of being used in cosmetic preparations.

## Claims

1. Surface-modified zinc oxide particles in which particle surfaces of zinc oxide particles are treated with a hydrolyzable surface treatment agent,
wherein a color difference ΔE before and after irradiation with simulated sunlight is 4.0 or lower.

2. The surface-modified zinc oxide particles according to claim 1,
wherein a total light transmittance at a wavelength of 360 nm is 48% or lower.

3. The surface-modified zinc oxide particles according to claim 1,
wherein a total light transmittance at a wavelength of 360 nm is 40% or lower.

4. The surface-modified zinc oxide particles according to claim 1,
wherein a total light transmittance at a wavelength of 360 nm after exposure to an environment of 85°C and 95 %RH for 48 hours is 40% or lower.

5. The surface-modified zinc oxide particles according to claim 2,
wherein a total light transmittance at a wavelength of 360 nm after exposure to the environment for 48 hours is 40% or lower.

6. The surface-modified zinc oxide particles according to any one of claims 1 to 5,
wherein a content of the surface treatment agent is 4% by mass or more and 20% by mass or less with respect to a total of the surface treatment agent and the zinc oxide particles.

7. The surface-modified zinc oxide particles according to any one of claims 1 to 6,
wherein the surface treatment agent is a hydrolyzed silane coupling agent having an alkoxy group.

8. A dispersion liquid comprising:
the surface-modified zinc oxide particles according to any one of claims 1 to 7; and
a dispersion medium.

9. A cosmetic preparation comprising:
the surface-modified zinc oxide particles according to any one of claims 1 to 7; and
a cosmetic base raw material.

10. A cosmetic preparation comprising:
the dispersion liquid according to claim 8; and
a cosmetic base raw material.

11. A method for producing surface-modified zinc oxide particles, which is a method for producing the surface-modified zinc oxide particles according to claim 1, the method comprising:
a first step of mixing a surface treatment agent and water to prepare a hydrolysate; and
a second step of mixing the hydrolysate obtained in the first step and zinc oxide particles to prepare a first mixture.

12. The method for producing surface-modified zinc oxide particles according to claim 11, further comprising:
a step of drying the first mixture obtained in the second step.

13. The method for producing surface-modified zinc oxide particles according to claim 11,
wherein the step of mixing the hydrolysate obtained in the first step and the mixture obtained in the step is repeated once or more.

14. The method for producing surface-modified zinc oxide particles according to claim 11, further comprising:
a third step of mixing the first mixture obtained in the second step and the hydrolysate obtained in the first step to prepare a second mixture containing the first mixture and the hydrolysate.

15. The method for producing surface-modified zinc oxide particles according to claim 14, further comprising:
a step of drying the second mixture obtained in the third step.
